# EUROPEAN PATENT SPECIFICATION

(11) **EP 3 747 370 B1**
(45) Date of publication and mention of the grant of the patent: **06.03.2024**
(21) Application number: 18903337.6
(22) Date of filing: 19.11.2018
(51) Int. Cl.: A61B 8/14, A61B 8/00

(54) **ULTRASOUND DIAGNOSTIC DEVICE AND CONTROL METHOD FOR ULTRASOUND DIAGNOSTIC DEVICE**
ULTRASCHALL-DIAGNOSEGERÄT UND STEUERVERFAHREN FÜR EIN ULTRASCHALL-DIAGNOSEGERÄT
DISPOSITIF DE DIAGNOSTIC ULTRASONORE ET PROCÉDÉ DE COMMANDE DESTINÉ À UN DISPOSITIF DE DIAGNOSTIC ULTRASONORE

(30) Priority: 31.01.2018 JP 2018015382; 30.05.2018 JP 2018103436
(43) Date of publication of application: 09.12.2020
(73) Proprietor: FUJIFILM Corporation, Tokyo 106-8620 (JP)
(72) Inventor: MATSUMOTO Tsuyoshi, kami-gun, Kanagawa 258-8538 (JP); INOUE Tomoki, kami-gun, Kanagawa 258-8538 (JP); EBATA Tetsurou, kami-gun, Kanagawa 258-8538 (JP)
(74) Representative: Dewhurst, Joseph Michael
(86) International application number: PCT/JP2018/042652
(87) International publication number: WO 2019/150715

(56) References cited:
- JP-A- 2009 056 125
- US-A1- 2010 049 050
- US-A1- 2017 105 701
- US-A1- 2017 273 668
- US-A1- 2017 296 148
- US-A1- 2017 360 403
- "Emergency Ultrasound Imaging Criteria Compendium ED - Hoekstra James; Qureshi Adnan", ANNALS OF EMERGENCY MEDICINE, LANSING, MI, US, vol. 48, no. 4, 1 October 2006 (2006-10-01), pages 487-510, XP005659496, ISSN: 0196-0644, DOI: 10.1016/J.ANNEMERGMED.2006.07.946
- DICKMAN E ET AL: "Clinician-performed abdominal sonography", EUROPEAN JOURNAL OF TRAUMA AND EMERGENCY SURGERY, SPRINGER BERLIN HEIDELBERG, BERLIN/HEIDELBERG, vol. 41, no. 5, 21 March 2015 (2015-03-21) , pages 481-492, XP035552908, ISSN: 1863-9933, DOI: 10.1007/S00068-015-0508-X [retrieved on 2015-03-21]
- KANG SEOK ET AL: "Ultrasound-Guided Cervical Nerve Root Block: Does Volume Affect the Spreading Pattern?", PAIN MEDICINE, vol. 17, no. 11, 23 March 2016 (2016-03-23), pages 1978-1984, XP055788640, US ISSN: 1526-2375, DOI: 10.1093/pm/pnw027 Retrieved from the Internet: URL:https://watermark.silverchair.com/pnw0 27.pdf?token=AQECAHi208BE49Ooan9kkhW_Ercy7 Dm3ZL_9Cf3qfKAc485ysgAAAsMwggK_BgkqhkiG9w0 BBwagggKwMIICrAIBADCCAqUGCSqGSIb3DQEHATAeB glghkgBZQMEAS4wEQQM3NnEUXGYW5fyobCkAgEQgII Cdo0thedweGl8SASV7fV_fI1i70_MEQmwJJWB3EG9v AZ8vBOgeeDeHsuGTSaAM9PtdgLrwhzQk8KkN_03OxU Q4BPCKlK5t>
- "Passages, Ultrasonic diagnosis, first edition, second printing" In: Japanese Society of Ultrasonic Medicine: "Ultrasonic diagnosis, first edition, second printing", 1 June 1989 (1989-06-01), Medical School, JP, XP009522162, ISBN: 4-260-10739-9 page 135, 407, 439, 469, 479, 493,
- Mori; Hideaki: "Ultrasound scan for gastrointestinal disorders", Journal of the Kyorin Medical Society, vol. 48, no. 1, 31 March 2017 (2017-03-31) , pages 29-38, XP055628573,

## Description

### BACKGROUND OF THE INVENTION

### 1. Field of the Invention

The present invention relates to an ultrasound diagnostic apparatus and a method for controlling the ultrasound diagnostic apparatus, and more specifically to an ultrasound diagnostic apparatus for guiding a user to operate an ultrasound probe and a method for controlling the ultrasound diagnostic apparatus.

### 2. Description of the Related Art

Ultrasound diagnostic apparatuses are known in the related art as apparatuses for obtaining an image of the inside of a subject. An ultrasound diagnostic apparatus typically includes an ultrasound probe including a vibrator array in which a plurality of elements are arrayed. While the ultrasound probe is in contact with the body surface of the subject, ultrasound beams are transmitted from the vibrator array to the inside of the subject, and ultrasound echoes from the subject are received by the vibrator array to acquire element data. Further, the ultrasound diagnostic apparatus electrically processes the obtained element data and generates an ultrasound image of the corresponding site of the subject.

Using such an ultrasound diagnostic apparatus, a user is able to observe sites in the subject. At this time, the user usually visually checks the ultrasound image to determine whether the intended site for observation is included in the ultrasound image, and such determination requires experience. Accordingly, various contrivances are made to the ultrasound diagnostic apparatus to easily detect the intended site.

For example, JP2015-171437A discloses a medical image processing apparatus that receives input of a plurality of ultrasound images acquired in advance and performs image analysis on each of the plurality of ultrasound images to automatically detect the intended site. US2017/296148A1 discloses a system for guiding a user to aim an ultrasound probe to capture one or more image frames of an organ of a patient, the system including: an image processor for capturing one or more image frames of a field of view of the ultrasound probe; detecting means for processing the one or more captured image frames to detect the presence of a predetermined anatomical feature of the patient having a known positional relationship with the organ; and guiding means for directing the user to adjust the aim and/ or position of the ultrasound probe so as to locate the organ within the field of view of the ultrasound probe based on the known positional relationship.

### SUMMARY OF THE INVENTION

In the subject, sites having similar structures are present, for example, the common bile duct and blood vessels. To observe a site such as the common bile duct having a structure similar to the structure of any other site such as blood vessels using an ultrasound diagnostic apparatus, a process flow that determines the operation procedure of an ultrasound probe is generally known. However, the intended site has a structure similar to the structure of any other site, and it is therefore difficult for even an experienced user to visually check an ultrasound image obtained in accordance with the process flow described above to identify the intended site, which is problematic.

The technique disclosed in JP2015-171437A, in which image analysis is performed on each of a large number of acquired ultrasound images to detect an intended site, makes it possible to detect a site having a structure similar to the structure of any other site. However, the calculation load required for the detection of the intended site is high, and an apparatus having high calculation performance is required to quickly detect the intended site. This apparatus is usually large-scale. For this reason, the medical image processing apparatus in JP2015-171437A may hinder the user from taking quick action in environments that require the user to take quick action, such as in emergency medical situations, and is thus unsuitable.

The present invention has been made to solve the problems of the related art described above, and it is an object of the present invention to provide an ultrasound diagnostic apparatus that enables easy and rapid detection of an intended site and a method for controlling the ultrasound diagnostic apparatus.

According to an aspect of the present invention, there is provided an ultrasound diagnostic apparatus as claimed in claim 1.

The peripheral site memory stores a plurality of peripheral sites effective to detect the target site and a determined detection order in which the plurality of peripheral sites are detected, and the operation guide unit guides the user to operate the ultrasound probe so as to sequentially detect the plurality of peripheral sites in accordance with the determined detection order.

Further, the operation guide unit can guide the user to operate the ultrasound probe so as to skip detection of some peripheral sites among the plurality of peripheral sites and detect a subsequent peripheral site on the basis of the recognition result obtained by the site recognition unit, or can guide the user to operate the ultrasound probe so as to change the determined detection order and detect the plurality of peripheral sites in the changed detection order.

Alternatively, the ultrasound diagnostic apparatus can further include an input unit that allows the user to perform an input operation.

The operation guide unit can guide the user to operate the ultrasound probe so as to skip detection of some peripheral sites among the plurality of peripheral sites and detect a subsequent peripheral site on the basis of correction information input by the user through the input unit, or can guide the user to operate the ultrasound probe so as to change the determined detection order and detect the plurality of peripheral sites in the changed detection order.

Alternatively, the operation guide unit may guide the user to operate the ultrasound probe so as to skip detection of some peripheral sites among the plurality of peripheral sites and detect a subsequent peripheral site on the basis of subject information concerning a state of the subject, which is input by the user through the input unit, or may guide the user to operate the ultrasound probe so as to change the determined detection order and detect the plurality of peripheral sites in the changed detection order.

Alternatively, when a determined amount of time elapses after the operation guide unit guides the user to operate the ultrasound probe so as to detect one peripheral site among the plurality of peripheral sites before the one peripheral site is detected, the operation guide unit may guide the user to operate the ultrasound probe so as to skip detection of the one peripheral site and detect a subsequent peripheral site, or may guide the user to operate the ultrasound probe so as to change the determined detection order and detect the plurality of peripheral sites in the changed detection order.

Further, the site recognition unit can recognize an imaged site of the subject on the basis of subject information concerning a state of the subject, which is input by the user through the input unit.

Further, the peripheral site memory can store, for each subject, the plurality of peripheral sites effective to detect the target site and the determined detection order, and the operation guide unit can guide the user to operate the ultrasound probe so as to sequentially detect the plurality of peripheral sites stored for each subject in accordance with the determined detection order stored for the subject.

The ultrasound diagnostic apparatus can further include a display unit, and the operation guide unit can display on the display unit a guide provided to the user to operate the ultrasound probe.

At this time, preferably, the ultrasound diagnostic apparatus further includes a contour generation unit that generates a contour of the at least one peripheral site recognized by the site recognition unit, the display unit displays the ultrasound image acquired by the image acquisition unit, and the contour of the peripheral site generated by the contour generation unit is displayed superimposed on the ultrasound image displayed on the display unit.

Alternatively, the ultrasound diagnostic apparatus can further include an audio generation unit, and the operation guide unit can guide the user to operate the ultrasound probe by generating audio from the audio generation unit.

Preferably, the target site is a common bile duct, and the least peripheral sites include a portal vein and a gallbladder.

Alternatively, preferably, the target site is an appendix, and the peripheral sites include an ascending colon, a cecum, and an ileum. Alternatively, the target site is a nerve root of a fifth cervical vertebra and a nerve root of a seventh cervical vertebra, and the peripheral site is a nerve root of a sixth cervical vertebra. Such an embodiment does not fall within the scope of the claims.

In another aspect of the present invention, there is provided a method for controlling an ultrasound diagnostic apparatus as claimed in claim 14.

In another aspect of the present invention, there is provided a processor for an ultrasound diagnostic apparatus as claimed in claim 15.

Further, the processor for an ultrasound diagnostic apparatus can be connected to the ultrasound probe via a network.

These features enable easy and rapid detection of the target site.

### BRIEF DESCRIPTION OF THE DRAWINGS

Fig. 1 is a block diagram illustrating a configuration of an ultrasound diagnostic apparatus according to Embodiment 1;
Fig. 2 is a block diagram illustrating an internal configuration of a receiving unit in Embodiment 1;
Fig. 3 is a block diagram illustrating an internal configuration of an image generation unit in Embodiment 1;
Fig. 4 is a flowchart illustrating the operation of the ultrasound diagnostic apparatus according to Embodiment 1;
Fig. 5 is a flowchart illustrating a specific example of the operation of the ultrasound diagnostic apparatus according to Embodiment 1;
Fig. 6 is a diagram illustrating an example of a guide marker in Embodiment 1;
Fig. 7 is a diagram illustrating another example of the guide marker in Embodiment 1;
Fig. 8 is a diagram illustrating still another example of the guide marker in Embodiment 1;
Fig. 9 is a diagram illustrating still another example of the guide marker in Embodiment 1;
Fig. 10 is a diagram illustrating an example of a guide marker in Embodiment 2;
Fig. 11 is a diagram illustrating another example of the guide marker in Embodiment 2;
Fig. 12 is a diagram illustrating still another example of the guide marker in Embodiment 2;
Fig. 13 is a diagram illustrating still another example of the guide marker in Embodiment 2;
Fig. 14 is a diagram schematically illustrating the fifth cervical vertebra, the sixth cervical vertebra, and the seventh cervical vertebra for which nerve roots detected in Embodiment 3 are located:
Fig. 15 is a diagram schematically illustrating the nerve root of the fifth cervical vertebra that is detected in Embodiment 3;
Fig. 16 is a flowchart illustrating the operation of an ultrasound diagnostic apparatus according to Embodiment 3;
Fig. 17 is a block diagram illustrating a configuration of an ultrasound diagnostic apparatus according to Embodiment 4;
Fig. 18 is a diagram illustrating an example of contour lines in Embodiment 4;
Fig. 19 is a diagram illustrating another example of the contour lines in Embodiment 4;
Fig. 20 is a diagram illustrating still another example of the contour lines in Embodiment 4;
Fig. 21 is a diagram illustrating still another example of the contour lines in Embodiment 4;
Fig. 22 is a flowchart illustrating the operation of an ultrasound diagnostic apparatus according to Embodiment 5;
Fig. 23 is a flowchart illustrating the operation of an ultrasound diagnostic apparatus according to Embodiment 6;
Fig. 24 is a block diagram illustrating a configuration of an ultrasound diagnostic apparatus according to Embodiment 7; and
Fig. 25 is a block diagram illustrating a configuration of an ultrasound diagnostic apparatus according to Embodiment 8.

### DESCRIPTION OF THE PREFERRED EMBODIMENTS

The following describes embodiments of this disclosure with reference to the accompanying drawings.

### Embodiment 1

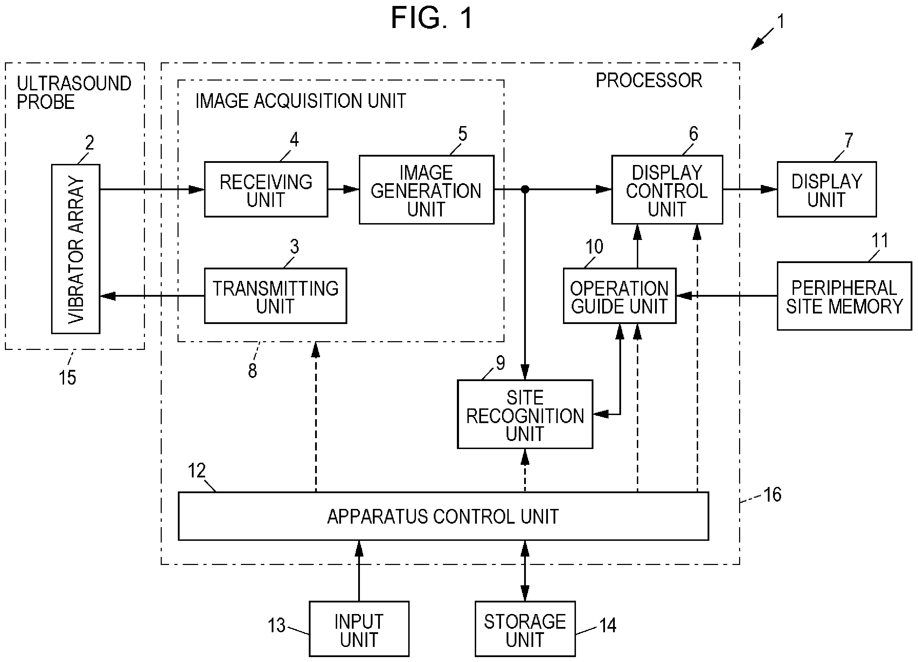

Fig. 1 illustrates a configuration of an ultrasound diagnostic apparatus 1 according to Embodiment 1. As illustrated in Fig. 1, the ultrasound diagnostic apparatus 1 includes a vibrator array 2, and the vibrator array 2 is connected to a transmitting unit 3 and a receiving unit 4. The receiving unit 4 is sequentially connected to an image generation unit 5, a display control unit 6, and a display unit 7. The transmitting unit 3, the receiving unit 4, and the image generation unit 5 constitute an image acquisition unit 8. The image generation unit 5 is further connected to a site recognition unit 9, and the site recognition unit 9 is connected to an operation guide unit 10. The site recognition unit 9 and the operation guide unit 10 are connected so as to enable two-way exchange of information. The operation guide unit 10 is further connected to a peripheral site memory 11 and the display control unit 6.

Further, the display control unit 6, the image acquisition unit 8, the site recognition unit 9, and the operation guide unit 10 are connected to an apparatus control unit 12, and the apparatus control unit 12 is connected to an input unit 13 and a storage unit 14. The apparatus control unit 12 and the storage unit 14 are connected so as to enable two-way exchange of information.

The vibrator array 2 is included in an ultrasound probe 15. The display control unit 6, the image acquisition unit 8, the site recognition unit 9, the operation guide unit 10, and the apparatus control unit 12 constitute a processor 16 for an ultrasound diagnostic apparatus.

The vibrator array 2 of the ultrasound probe 15 illustrated in Fig. 1 has a plurality of vibrators that are arrayed one-dimensionally or two-dimensionally. Each of these vibrators transmits an ultrasound wave in accordance with a drive signal supplied from the transmitting unit 3 and outputs a reception signal upon receipt of an ultrasound echo from the subject. Each vibrator is constructed by, for example, forming electrodes at both ends of a piezoelectric body composed of a piezoelectric ceramic typified by PZT (Lead Zirconate Titanate), a polymeric piezoelectric element typified by PVDF (Poly Vinylidene Di Fluoride), a piezoelectric single crystal typified by PMN-PT (Lead Magnesium Niobate-Lead Titanate), or the like.

The transmitting unit 3 of the image acquisition unit 8 includes, for example, a plurality of pulse generators, and supplies to the plurality of vibrators of the vibrator array 2 respective drive signals whose amounts of delay are adjusted so that the ultrasound waves transmitted from the plurality of vibrators form an ultrasound beam on the basis of a transmission delay pattern selected in accordance with a control signal from the apparatus control unit 12. In this manner, when a pulsed or continuous-wave voltage is applied to the electrodes of the plurality of vibrators of the vibrator array 2, the piezoelectric bodies expand and contract. Pulsed or continuous-wave ultrasound waves are generated from the respective vibrators, and a composite wave of these ultrasound waves forms an ultrasound beam.

The transmitted ultrasound beam is reflected from, for example, a target such as a site of the subject and propagates toward the vibrator array 2 of the ultrasound probe 15. The ultrasound echo propagating toward the vibrator array 2 in this manner is received by the respective vibrators of the vibrator array 2. At this time, upon receipt of the propagating ultrasound echo, the respective vibrators of the vibrator array 2 expand and contract to generate electrical signals, and these electrical signals are output to the receiving unit 4.

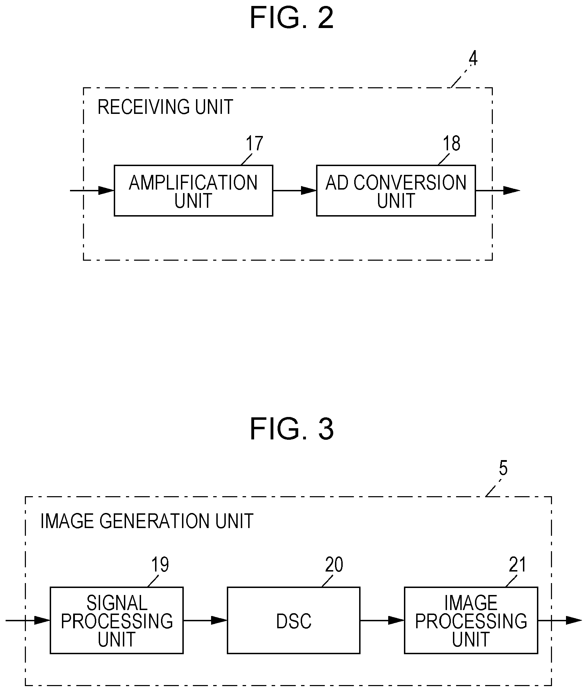

The receiving unit 4 of the image acquisition unit 8 performs processing of the reception signals output from the vibrator array 2 in accordance with a control signal from the apparatus control unit 12. As illustrated in Fig. 2, the receiving unit 4 has a configuration in which an amplification unit 17 and an AD (Analog Digital) conversion unit 18 are connected in series. The amplification unit 17 amplifies the reception signals input from the respective elements of the vibrator array 2 and transmits the amplified reception signals to the AD conversion unit 18. The AD conversion unit 18 converts the reception signals transmitted from the amplification unit 17 into digital data and sends the data to the image generation unit 5 of the image acquisition unit 8.

As illustrated in Fig. 3, the image generation unit 5 of the image acquisition unit 8 has a configuration in which a signal processing unit 19, a DSC (Digital Scan Converter) 20, and an image processing unit 21 are connected in series. The signal processing unit 19 performs reception focus processing in which the pieces of data of the reception signals are given respective delays on the basis of a reception delay pattern selected in accordance with a control signal from the apparatus control unit 12 and are added together (phasing addition). Through the reception focus processing, a sound ray signal in which the focus of the ultrasound echo is narrowed to a single scan line is generated. Further, the signal processing unit 19 corrects the generated sound ray signal for attenuation caused by the propagation distance in accordance with the depth of the position at which the ultrasound wave is reflected, and then performs envelope detection processing to generate a B-mode image signal indicating tissue in the subject. The B-mode image signal generated in this way is output to the DSC 20.

The DSC 20 of the image generation unit 5 performs raster conversion to convert the B-mode image signal into an image signal based on a typical television signal scanning method to generate an ultrasound image. The image processing unit 21 of the image generation unit 5 performs various necessary image processing operations, such as brightness correction, gradation correction, sharpness correction, and color correction, on the image data obtained by the DSC 20, and then outputs the ultrasound image to the display control unit 6 and the site recognition unit 9.

The site recognition unit 9 of the processor 16 performs image analysis on the ultrasound image acquired by the image acquisition unit 8 to recognize the imaged site of the subject. At this time, for example, the site recognition unit 9 can store in advance typical pattern data as a template, search through an image using the template to calculate a degree of similarity to the pattern data, and identify a location having a maximum degree of similarity greater than or equal to a threshold value as a location in which the measurement target is present to recognize the imaged site. The degree of similarity can be calculated using, as well as simple template matching, for example, a machine learning technique as described in Csurka et al.: Visual Categorization with Bags of Keypoints, Proc. of ECCV Workshop on Statistical Learning in Computer Vision, pp. 59-74 (2004), a typical image recognition technique based on deep learning as described in Krizhevsk et al.: ImageNet Classification with Deep Convolutional Neural Networks, Advances in Neural Information Processing Systems 25, pp. 1106-1114 (2012), or the like.

In the subject, sites having similar structures are present, for example, the common bile duct and blood vessels. In this manner, the site such as the common bile duct has a structure similar to the structure of any other site such as blood vessels and may be difficult to accurately identify even when an experienced user performs observation. The peripheral site memory 11 of the ultrasound diagnostic apparatus 1 stores the peripheral sites effective to detect a target site. The target site is difficult to accurately identify and is to be observed. When a plurality of peripheral sites effective to detect the target site are stored, the peripheral site memory 11 also stores a determined detection order in which the plurality of peripheral sites are detected.

Examples of the peripheral site memory 11 include recording media, such as an HDD (Hard Disc Drive), an SSD (Solid State Drive), an FD (Flexible Disc), an MO disc (MagnetoOptical disc), an MT (Magnetic Tape), a RAM (Random Access Memory), a CD (Compact Disc), a DVD (Digital Versatile Disc), an SD card (Secure Digital card), and a USB memory (Universal Serial Bus memory), and a server.

During detection of the target site, the operation guide unit 10 of the processor 16 guides the user to operate the ultrasound probe 15 so as to detect the peripheral sites stored in the peripheral site memory 11, and further guides the user to operate the ultrasound probe 15 so as to detect the target site on the basis of the recognition result obtained by the site recognition unit 9 of the processor 16. The ultrasound diagnostic apparatus 1 according to Embodiment 1 can quickly detect the target site using the operation guide unit 10, which will be described in detail below.

The apparatus control unit 12 of the processor 16 controls each of the units of the ultrasound diagnostic apparatus 1 in accordance with a program stored in advance in the storage unit 14 or the like and in accordance with the user's operation through the input unit 13.

The display control unit 6 of the processor 16 performs, under control of the apparatus control unit 12, predetermined processing on the ultrasound image generated by the image generation unit 5 of the image acquisition unit 8 and causes the display unit 7 to display the ultrasound image.

The display unit 7 of the ultrasound diagnostic apparatus 1 displays an image under control of the display control unit 6. The display unit 7 includes, for example, a display device such as an LCD (Liquid Crystal Display).

The input unit 13 of the ultrasound diagnostic apparatus 1 allows the user to perform an input operation, and is configured to include a keyboard, a mouse, a trackball, a touchpad, a touch panel, and the like.

The storage unit 14 stores an operation program and the like for the ultrasound diagnostic apparatus 1. Like the peripheral site memory 11 of the ultrasound diagnostic apparatus 1, examples of the storage unit 14 include recording media, such as an HDD, an SSD, an FD, an MO disc, an MT, a RAM, a CD, a DVD, an SD card, and a USB memory, and a server.

The processor 16 having the display control unit 6, the image acquisition unit 8, the site recognition unit 9, the operation guide unit 10, and the apparatus control unit 12 is constituted by a CPU (Central Processing Unit) and a control program for causing the CPU to perform various processing operations, or may be configured using a digital circuit. The display control unit 6, the image acquisition unit 8, the site recognition unit 9, the operation guide unit 10, and the apparatus control unit 12 may be configured to be partially or entirely integrated into a single CPU.

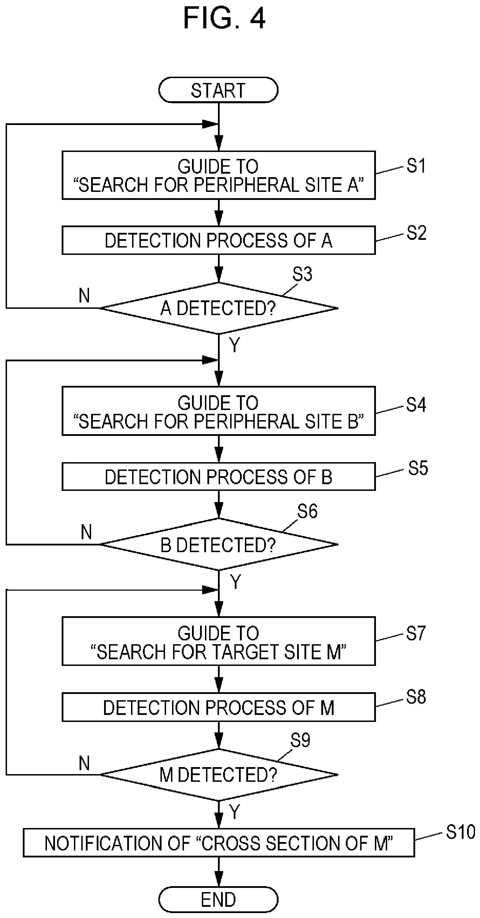

Next, the operation of the ultrasound diagnostic apparatus 1 according to Embodiment 1 will be described in detail with reference to a flowchart illustrated in Fig. 4. The flowchart illustrated in Fig. 4 depicts the operation of the ultrasound diagnostic apparatus 1 for detecting a target site M. The target site M can be set by, for example, being input by the user through the input unit 13. The peripheral site memory 11 is assumed to store peripheral sites A and B as peripheral sites effective to detect the target site M, and to further store a detection order such that the detection process of the peripheral site A is performed and then the detection process of the peripheral site B is performed.

First, in step S1, the operation guide unit 10 guides the user to search for the peripheral site A. At this time, for example, the operation guide unit 10 can display a guide marker to search for the peripheral site A on the display unit 7 through the display control unit 6.

When a guide to search for the peripheral site A is provided in step S1, the user operates the ultrasound probe 15 so that the peripheral site A is detected in accordance with the guide provided by the operation guide unit 10. In this manner, in the state where the ultrasound probe 15 is being operated by the user, in step S2, the site recognition unit 9 performs the detection process of the peripheral site A. At this time, although not illustrated, the site recognition unit 9 can recognize at least one auxiliary site effective to detect the peripheral site A, and can detect the peripheral site A in consideration of this recognition result.

Then, in step S3, the operation guide unit 10 determines whether the peripheral site A has been detected. If the peripheral site A has not been detected, the process returns to step S1, and the operation guide unit 10 provides a guide to search for the peripheral site A. After the detection process of the peripheral site A is performed in step S2, the determination of step S3 is performed. In this way, the processing of steps S1 to S3 is repeatedly performed until the peripheral site A is detected.

If it is determined in step S3 that the peripheral site A has been detected, the process proceeds to step S4, and the operation guide unit 10 provides a guide to search for the peripheral site B. At this time, the operation guide unit 10 can display a guide marker to search for the peripheral site B on the display unit 7. In addition, the operation guide unit 10 can display a guide marker to guide the movement direction, orientation, inclination, and the like of the ultrasound probe 15 on the display unit 7 to detect the peripheral site B on the basis of the position of the detected peripheral site A.

When a guide to search for the peripheral site B is provided in step S4, the user operates the ultrasound probe 15 so that the peripheral site B is detected in accordance with the guide provided by the operation guide unit 10. In this manner, in the state where the ultrasound probe 15 is being operated by the user, in step S5, the site recognition unit 9 performs the detection process of the peripheral site B. At this time, the site recognition unit 9 may detect the peripheral site B in consideration of the recognition result of the peripheral site A, although not illustrated. For example, a relative positional relationship between the peripheral sites A and B may be stored in the peripheral site memory 11 or the like in advance to allow the site recognition unit 9 to detect the peripheral site B in consideration of the relative positional relationship between the peripheral site A and the peripheral site B.

Then, in step S6, the operation guide unit 10 determines whether the peripheral site B has been detected. If the peripheral site B has not been detected, the process returns to step S4, and the operation guide unit 10 provides a guide to search for the peripheral site B. After the detection process of the peripheral site B is performed in step S5, the determination of step S6 is performed. In this way, the processing of steps S4 to S6 is repeatedly performed until the peripheral site B is detected.

If it is determined in step S6 that the peripheral site B has been detected, the process proceeds to step S7, and the operation guide unit 10 provides a guide to search for the target site M. At this time, the operation guide unit 10 can display a guide marker to search for the target site M on the display unit 7. In addition, the operation guide unit 10 can display a guide marker to guide the movement direction, orientation, inclination, and the like of the ultrasound probe 15 on the display unit 7 to detect the target site M on the basis of the positions of the detected peripheral sites A and B.

When a guide to search for the target site M is provided in step S7, the user operates the ultrasound probe 15 so that the target site M is detected in accordance with the guide provided by the operation guide unit 10. In this manner, in the state where the ultrasound probe 15 is being operated by the user, in step S8, the site recognition unit 9 performs the detection process of the target site M. At this time, the site recognition unit 9 may detect the target site M in consideration of the recognition results of the peripheral sites A and B, although not illustrated. For example, a relative positional relationship between the target site M and the peripheral sites A and B may be stored in the peripheral site memory 11 or the like in advance to allow the site recognition unit 9 to detect the target site M in consideration of the relative positional relationship between the target site M and the peripheral sites A and B.

Then, in step S9, the operation guide unit 10 determines whether the target site has been detected. If the target site M has not been detected, the process returns to step S7, and the operation guide unit 10 provides a guide to search for the target site M. After the detection process of the target site M is performed in step S8, the determination of step S9 is performed. In this way, the processing of steps S7 to S9 is repeatedly performed until the target site M is detected.

If it is determined in step S9 that the target site M has been detected, the process proceeds to step S 10. In step S10, the operation guide unit 10 notifies the user that the cross section of the target site M is displayed on the display unit 7. Then, the operation of the ultrasound diagnostic apparatus 1 according to Embodiment 1 ends.

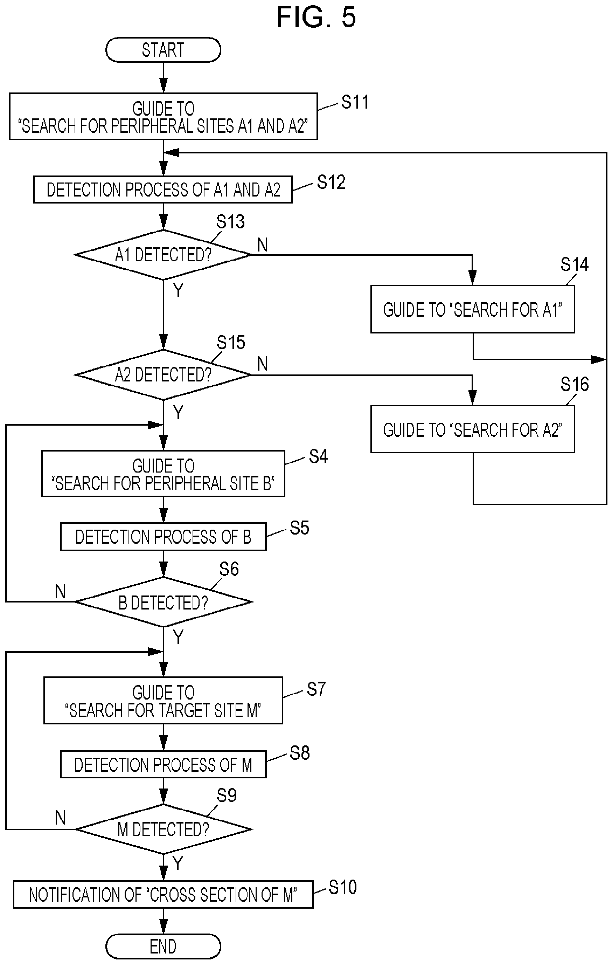

Next, a specific example of the operation of the ultrasound diagnostic apparatus 1 according to Embodiment 1 of the present invention will be introduced with reference to Fig. 5 to Fig. 8. The flowchart illustrated in Fig. 5 depicts the operation of the ultrasound diagnostic apparatus 1 for detecting the common bile duct as the target site M. It is assumed here that the peripheral site memory 11 stores a short-axis view of the portal vein, a long-axis view of the gallbladder, and a long-axis view of the portal vein as peripheral sites A1, A2, and B effective to detect the common bile duct, respectively, and further stores a detection order such that the detection process of the peripheral site A1 and the detection process of the peripheral site A2 are performed and then the detection of the peripheral site B is performed.

The short-axis view of the portal vein represents a transverse cross-sectional image of the cross section of the portal vein taken along a plane perpendicular to the central axis of the portal vein, although not illustrated. The long-axis view of the portal vein represents a longitudinal cross-sectional image of the cross section of the portal vein taken along the central axis of the portal vein. Also, the long-axis view of the gallbladder represents a longitudinal cross-sectional image of the cross section of the gallbladder taken along the central axis of the gallbladder in a manner similar to that for the long-axis view of the portal vein.

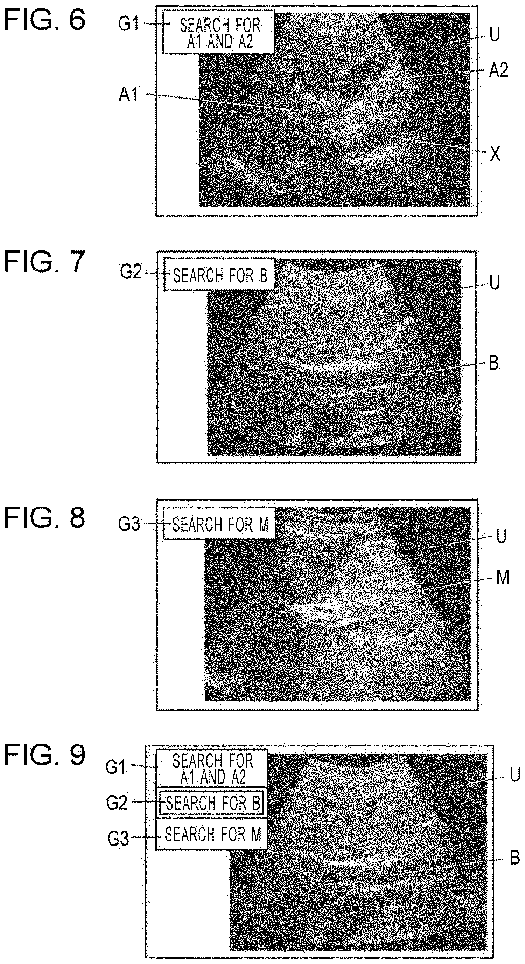

First, in step S11, the operation guide unit 10 guides the user to search for the peripheral site A1, which is the short-axis view of the portal vein, and the peripheral site A2, which is the long-axis view of the gallbladder. At this time, for example, as illustrated in Fig. 6, the operation guide unit 10 can display a guide marker G1 to search for the peripheral sites A1 and A2 on the display unit 7 together with an ultrasound image U. As illustrated in Fig. 6, the guide marker G1 may include a text, or may include a guide image representing a guide for the user.

When a guide to search for the peripheral sites A1 and A2 is provided in step S11, the user operates the ultrasound probe 15 so that the peripheral sites A1 and A2 are detected in accordance with the guide provided by the operation guide unit 10. In this manner, in the state where the ultrasound probe 15 is being operated by the user, in step S12, the site recognition unit 9 performs the detection process of the peripheral sites A1 and A2. At this time, for example, as illustrated in Fig. 6, the site recognition unit 9 can recognize the inferior vena cava or the like as an auxiliary site X and can detect the peripheral sites A1 and A2 in consideration of the recognition result.

Then, in step S13, the operation guide unit 10 determines whether the peripheral site A1 has been detected. If the peripheral site A1 has not been detected, the process proceeds to step S14, and the operation guide unit 10 provides a guide to search for the peripheral site A1. When a guide to search for the peripheral site A1 is provided in step S14, the process returns to step S12, and the detection process of the peripheral sites A1 and A2 is performed. Then, the determination of step S 13 is performed. In this way, the processing of steps S12 to S 14 is repeatedly performed until the peripheral site A1 is detected.

If it is determined in step S13 that the peripheral site A1 has been detected, the process proceeds to step S15. In step S15, the operation guide unit 10 determines whether the peripheral site A2 has been detected. If the peripheral site A2 has not been detected, the process proceeds to step S16, and the operation guide unit 10 provides a guide to search for the peripheral site A2. When a guide to search for the peripheral site A2 is provided in step S16, the process returns to step S12, and the detection process of the peripheral sites A1 and A2 is performed. Then, in step S13, it is determined whether the peripheral site A1 has been detected. Since the peripheral site A1 has already been detected, the process proceeds to step S15, and it is determined whether the peripheral site A2 has been detected. In this way, the processing of steps S12, S13, S15, and S16 is repeatedly performed until the peripheral site A2 is detected.

The subsequent processing of steps S4 to S10 is similar to that of steps S4 to S10 in the flowchart illustrated in Fig. 4. That is, first, in step S4, the operation guide unit 10 guides the user to search for the peripheral site B, which is the long-axis view of the portal vein. At this time, for example, as illustrated in Fig. 7, the operation guide unit 10 can display a guide marker G2 to search for the peripheral site B on the display unit 7 together with the ultrasound image U.

When a guide to search for the peripheral site B is provided in step S4, then, in step S5, the site recognition unit 9 performs the detection process of the peripheral site B. Then, in step S6, the operation guide unit 10 determines whether the peripheral site B has been detected. If the peripheral site B has not been detected, the process returns to step S4, and a guide to search for the peripheral site B is provided. Then, in steps S5 and S6, the detection process of the peripheral site B is performed, and it is determined whether the peripheral site B has been detected. In this way, the processing of steps S4 to S6 is repeatedly performed until the peripheral site B is detected. If the peripheral site B has been detected, the process proceeds to step S7.

In step S7, the operation guide unit 10 guides the user to search for the target site M, which is the common bile duct. At this time, for example, as illustrated in Fig. 8, the operation guide unit 10 can display a guide marker G3 to search for the target site M on the display unit 7 together with the ultrasound image U.

When a guide to search for the target site M is provided in step S7, then, in step S8, the site recognition unit 9 performs the detection process of the target site M. Then, in step S9, the operation guide unit 10 determines whether the target site M has been detected. If the target site M has not been detected, the process returns to step S7, and a guide to search for the target site M is provided. Then, in steps S8 and S9, the detection process of the target site M is performed, and it is determined whether the target site M has been detected. In this way, the processing of steps S7 to S9 is repeatedly performed until the target site M is detected. If the target site M has been detected, the process proceeds to step S10.

In step S10, the operation guide unit 10 notifies the user that the cross section of the target site M is displayed on the display unit 7. Then, the operation of the ultrasound diagnostic apparatus 1 for detecting the common bile duct, which is the target site M, ends.

As described above, the ultrasound diagnostic apparatus 1 according to Embodiment 1 guides a user to operate the ultrasound probe 15 so as to detect the peripheral sites effective to detect the target site M, and guides the user to operate the ultrasound probe 15 so as to detect the target site M on the basis of the recognition result of the peripheral sites. This eliminates the need to perform an unnecessary image recognition process and enables easy and rapid detection of the target site M with the reduced calculation load on the ultrasound diagnostic apparatus 1.

It is known that, when detecting a site such as the common bile duct having a structure similar to the structure of a site such as blood vessels in which blood flows, an ultrasound diagnostic apparatus of the related art performs so-called Doppler measurement to distinguish between the site such as blood vessels in which blood flows and the site such as the common bile duct. Typically, the processing to be performed by the ultrasound diagnostic apparatus needs to be switched from processing for acquiring a B-mode image representing a tomographic image of the subject to processing for performing Doppler measurement, which is time-consuming for the user. In contrast, the ultrasound diagnostic apparatus 1 according to Embodiment 1 eliminates the need to perform Doppler measurement to detect the target site M and enables more rapid detection of the target site M.

The ultrasound diagnostic apparatus 1 may finish the operation at the point in time when the processing of step S7 in the flowchart illustrated in Fig. 4 and the flowchart illustrated in Fig. 5 is complete, that is, at the point in time when the operation guide unit 10 provides a guide to search for the target site M, and may stop the processing of steps S8 to S10. In this case, the user may visually check the ultrasound image displayed on the display unit 7 to determine whether the ultrasound image contains the cross section of the target site M. Even in this case, the operation guide unit 10 guides the user to operate the ultrasound probe 15 so as to detect the peripheral sites effective to detect the target site M, and guides the user to operate the ultrasound probe 15 so as to detect the target site M on the basis of the recognition result obtained by the site recognition unit 9. This allows the user to rapidly identify the target site M.

In the flowchart illustrated in Fig. 5, the detection order of the peripheral sites A1 and A2 is determined so that the peripheral site A1 and the peripheral site A2 are detected simultaneously. In this case, there is no limit on which operation to perform first out of the determination of whether the peripheral site A1 has been detected and the determination of whether the peripheral site A2 has been detected. For example, in the flowchart illustrated in Fig. 5, the processing of step S13 may be performed after the processing of step S15 is performed.

In Embodiment 1, the transmitting unit 3 and the receiving unit 4 are included in the image acquisition unit 8 of the processor 16. Alternatively, the transmitting unit 3 and the receiving unit 4 may be included in the ultrasound probe 15. In this case, the transmitting unit 3 and the receiving unit 4 included in the ultrasound probe 15 and the image generation unit 5 included in the processor 16 constitute the image acquisition unit 8.

In Embodiment 1, furthermore, as illustrated in the flowcharts in Fig. 4 and Fig. 5, a plurality of peripheral sites effective to detect the target site M are stored in the peripheral site memory 11, by way of example. A single peripheral site effective to detect the target site M may be stored in the peripheral site memory 11 instead. In this case, the operation guide unit 10 guides the user to operate the ultrasound probe 15 so as to detect the target site M on the basis of the recognition result of the single peripheral site.

Furthermore, the peripheral site memory 11 may store a plurality of candidate peripheral sites that may be one peripheral site effective to detect the target site M, and the user may select one of the candidate peripheral sites and use the candidate peripheral site as a peripheral site for detecting the target site M. For example, the peripheral site memory 11 can store sites C and D as candidates of one peripheral site, and the user can select any one of the sites C and D through the input unit 13 as a peripheral site. In this case, the operation guide unit 10 guides the user to operate the ultrasound probe 15 so that the peripheral site selected by the user through the input unit 13 from among the sites C and D is detected, and guides the user to operate the ultrasound probe 15 so as to detect the target site M on the basis of the recognition result of the selected peripheral site.

Likewise, the peripheral site memory 11 may store of a plurality of sets of candidate peripheral sites that may be a plurality of peripheral sites effective to detect the target site M, and the user may select one set of candidate peripheral sites from among the sets of candidate peripheral sites and use the set of candidate peripheral sites as a plurality of peripheral sites for detecting the target site M. For example, the peripheral site memory 11 can store, as a plurality of sets of candidate peripheral sites, a set of sites A, B, and C and a set of sites B, C, and D. In this case, for example, in response to the user selecting one of the plurality of sets of candidate peripheral sites through the input unit 13, the operation guide unit 10 can guide the user to operate the ultrasound probe 15 so as to detect the target site M in accordance with the selected set of peripheral sites. Accordingly, the ultrasound diagnostic apparatus 1 can guide the user in accordance with a more suitable set of peripheral sites for the state of the subject and the like. This enables more rapid detection of the target site M.

Further, the peripheral site memory 11 may store, for each subject, a plurality of peripheral sites effective to detect the target site M and the detection order thereof. In this case, for example, in response to the user inputting identification information for identifying the subject through the input unit 13, the operation guide unit 10 guides the user to operate the ultrasound probe 15 so as to sequentially detect the plurality of peripheral sites stored for the subject identified by the identification information in accordance with the detection order stored for the subject. Accordingly, the target site M is detected in accordance with peripheral sites and detection order suitable for a subject. This enables more rapid detection of the target site M.

Some sites among a plurality of sites in the subject may change in size, shape, and the like depending on the state of the subject. For example, the gallbladder immediately after the subject has had a meal typically contracts more than the gallbladder before the subject has a meal. The site recognition unit 9 of the processor 16 can recognize an imaged site even if the size, shape, and the like of the site change depending on the state of the subject. For example, the site recognition unit 9 changes the algorithm for recognizing an imaged site of the subject, such as the template, on the basis of subject information concerning the state of the subject input by the user through the input unit 13, such as the pre-prandial state or the postprandial state, to recognize a site whose size, shape, and the like are changed depending on the state of the subject. For example, when subject information indicating that the subject remains in the postprandial state is input by the user through the input unit 13, the site recognition unit 9 can recognize the gallbladder by using the algorithm corresponding to a contracted gallbladder. The subject information may include information on the subject, such as the height, weight, and sex.

In this way, the site recognition unit 9 recognizes a site whose size, shape, and the like are changed depending on the state of the subject to smoothly guide the user with reduced likelihood that the detection of the peripheral sites effective to detect the target site M will fail. Accordingly, the ultrasound diagnostic apparatus 1 can more quickly detect the target site M.

In the foregoing description, when the operation guide unit 10 guides the user to operate the ultrasound probe 15, as illustrated in Fig. 6 to Fig. 8, the guide markers G1, G2, and G3 are displayed as example guides on the display unit 7 together with the ultrasound image U. For example, as illustrated in Fig. 9, the series of guide markers G1 to G3 to be displayed on the display unit 7 to detect the target site M can be displayed together with the ultrasound image U, and any one of the guide markers G1 to G3 can be highlighted in accordance with the progression of the guide provided by the operation guide unit 10. In the example illustrated in Fig. 9, among the guide markers G1 to G3, the guide marker G2 for searching for the peripheral site B is highlighted. As used here, the term "highlighting" refers to displaying a specific guide marker in a different display style from other guide markers, such as displaying the specific guide marker in different color from other guide markers or making the frame of the specific guide marker using a different type of line from the frames of other guide markers. This allows the user to easily understand the content of the series of guides provided by the operation guide unit 10 and the progression of the guides provided by the operation guide unit 10. Thus, the user is able to more smoothly operate the ultrasound probe 15 to detect the target site M.

As in the example illustrated in Fig. 9, when a series of guide markers for detecting the target site M is displayed on the display unit 7, a guide marker regarding a site already detected by the site recognition unit 9 may be changed to a guide marker indicating that detection has been carried out. For example, in the example illustrated in Fig. 9, since the peripheral sites A1 and A2 have already been detected, the guide marker G1 to "search for A1 and A2" can be changed to a guide marker indicating "A1 and A2 have already been detected", although not illustrated. This allows the user to more clearly understand the progression of the guides provided by the operation guide unit 10.

Further, although not illustrated, the operation guide unit 10 can display, near a site recognized by the site recognition unit 9, a text, an image, and the like indicating the name of the site to be superimposed on the ultrasound image U displayed on the display unit 7 such that, for example, a text that reads "portal vein" is displayed near a short-axis view of the portal vein, which is a peripheral site in the ultrasound image U. At this time, for example, the operation guide unit 10 can also display a mark such as an arrow extending from the text, image, and the like indicating the name of the site toward the site recognized by the site recognition unit 9, to be superimposed on the ultrasound image U. This allows the user to clearly understand the peripheral site, the auxiliary site, and the target site M in the ultrasound image U and to more easily and rapidly detect the target site M.

Further, although not illustrated, when guiding a user to operate the ultrasound probe 15 so as to detect a peripheral site effective to detect the target site M, the operation guide unit 10 can display a reference image and the currently acquired ultrasound image side by side. The reference image is an example reference image including the site currently being detected. Examples of the reference image include a previously acquired ultrasound image of the subject currently being subjected to ultrasound diagnosis, an ultrasound image of any other subject, and an ultrasound image appearing in an article such as a reference book. In this manner, the user operating the ultrasound probe 15 while referring to the reference image is able to more smoothly operate the ultrasound probe 15 in accordance with the guide provided by the operation guide unit 10.

Further, in the ultrasound diagnostic apparatus 1, although not illustrated, the ultrasound probe 15 can include an attitude angle detection sensor configured to include a sensor such as an acceleration sensor, a gyro-sensor, a magnetic sensor, or a GPS (Global Positioning System) sensor. The attitude angle detection sensor is a sensor that detects an attitude angle indicating the inclination of the ultrasound probe 15 and its direction. This enables the operation guide unit 10 to guide the user to operate the ultrasound probe 15 on the basis of the attitude angle of the ultrasound probe 15 obtained when a peripheral site effective to detect the target site M is detected.

For example, the operation guide unit 10 may alert the user when the current attitude angle of the ultrasound probe 15 greatly deviates from the attitude angle of the ultrasound probe 15 at which an optimum tomographic image of the subject to detect the target site M is obtained. Further, for example, the operation guide unit 10 may guide a specific direction, angle, and the like for operating the ultrasound probe 15 so as to make the current attitude angle of the ultrasound probe 15 close to the attitude angle of the ultrasound probe 15 at which an optimum tomographic image of the subject to detect the target site M is obtained.

### Embodiment 2

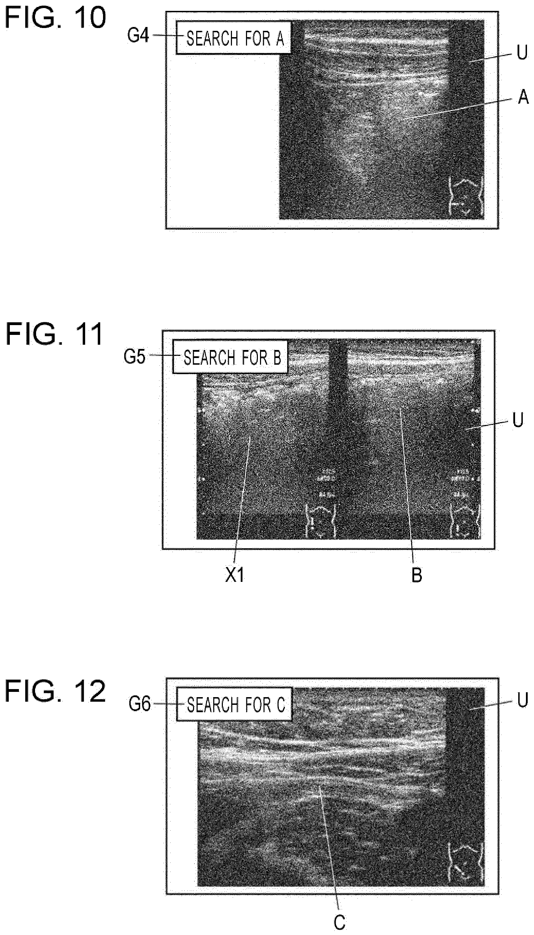

Embodiment 1 provides an example in which the common bile duct is detected as a specific example of the target site M. The present disclosure is also applicable to the detection of any other site. In Embodiment 2, the operation of the ultrasound diagnostic apparatus 1 for detecting the appendix as a specific example of the target site M different from the common bile duct will be introduced with reference to Fig. 10 to Fig. 13. It is assumed here that the peripheral site memory 11 stores a short-axis view of the ascending colon as a peripheral site A effective to detect the appendix, a long-axis view of the cecum as a peripheral site B, and a long-axis view of the ileum as a peripheral site C, and further stores a detection order such that the peripheral sites are detected in the order of the peripheral site A, the peripheral site B, and the peripheral site C.

The short-axis view of the ascending colon represents a transverse cross-sectional image of the cross section of the ascending colon taken along a plane perpendicular to the central axis of the ascending colon, the long-axis view of the cecum represents a longitudinal cross-sectional image of the cross section of the cecum taken along the central axis of the cecum, and the long-axis view of the ileum represents a longitudinal cross-sectional image of the cross section of the ileum taken along the central axis of the ileum.

First, the operation guide unit 10 guides the user to search for the peripheral site A, which is the short-axis view of the ascending colon. At this time, for example, as illustrated in Fig. 10, the operation guide unit 10 can display a guide marker G4 to search for the peripheral site A on the display unit 7 together with the ultrasound image U. As illustrated in Fig. 10, the guide marker G4 may include a text, or may include a guide image representing a guide for the user.

When a guide to search for the peripheral site A is provided, the user operates the ultrasound probe 15 so that the peripheral site A is detected in accordance with the guide provided by the operation guide unit 10. In this manner, in the state where the ultrasound probe 15 is being operated by the user, the site recognition unit 9 performs the detection process of the peripheral site A.

Then, the operation guide unit 10 determines whether the peripheral site A has been detected. If the peripheral site A has not been detected, the operation guide unit 10 again provides a guide to search for the peripheral site A, and the site recognition unit 9 performs the detection process of the peripheral site A. In this way, a guiding process using the operation guide unit 10 and the detection process of the peripheral site A using the site recognition unit 9 are repeatedly performed until the peripheral site A is detected.

If the operation guide unit 10 determines that the peripheral site A has been detected, for example, as illustrated in Fig. 11, the operation guide unit 10 guides the user to search for the peripheral site B, which is the long-axis view of the cecum. At this time, the operation guide unit 10 can display a guide marker G5 to search for the peripheral site B on the display unit 7 together with the ultrasound image U. The operation guide unit 10 can provide a specific instruction to the user, such as displaying in the guide marker G5 a message that prompts the user to rotate the orientation of the ultrasound probe 15 by 90 degrees to acquire a tomographic image including the peripheral site B.

When a guide to search for the peripheral site B is provided, the user operates the ultrasound probe 15 so that the peripheral site B is detected in accordance with the guide provided by the operation guide unit 10. In this manner, in the state where the ultrasound probe 15 is being operated by the user, the site recognition unit 9 performs the detection process of the peripheral site B. At this time, for example, as illustrated in Fig. 11, the site recognition unit 9 can recognize a long-axis view of the ascending colon or the like as an auxiliary site X1 and can detect the peripheral site B in consideration of the recognition result. The long-axis view of the ascending colon represents a longitudinal cross-sectional image of the cross section of the ascending colon taken along the central axis of the ascending colon.

Then, the operation guide unit 10 determines whether the peripheral site B has been detected. If the peripheral site B has not been detected, the operation guide unit 10 again provides a guide to search for the peripheral site B, and the site recognition unit 9 performs the detection process of the peripheral site B. In this way, a guiding process using the operation guide unit 10 and the detection process of the peripheral site B using the site recognition unit 9 are repeatedly performed until the peripheral site B is detected.

If the operation guide unit 10 determines that the peripheral site B has been detected, for example, as illustrated in Fig. 12, the operation guide unit 10 guides the user to search for the peripheral site C, which is the long-axis view of the ileum. At this time, the operation guide unit 10 can display a guide marker G6 to search for the peripheral site C on the display unit 7 together with the ultrasound image U. The operation guide unit 10 can provide a specific instruction to the user, such as displaying in the guide marker G6 a message that prompts the user to incline the ultrasound probe 15 leftward to acquire a tomographic image including the peripheral site C.

When a guide to search for the peripheral site C is provided, the user operates the ultrasound probe 15 so that the peripheral site C is detected in accordance with the guide provided by the operation guide unit 10. In this manner, in the state where the ultrasound probe 15 is being operated by the user, the site recognition unit 9 performs the detection process of the peripheral site C.

Then, the operation guide unit 10 determines whether the peripheral site C has been detected. If the peripheral site C has not been detected, the operation guide unit 10 again provides a guide to search for the peripheral site C, and the site recognition unit 9 performs the detection process of the peripheral site C. In this way, a guiding process using the operation guide unit 10 and the detection process of the peripheral site C using the site recognition unit 9 are repeatedly performed until the peripheral site C is detected.

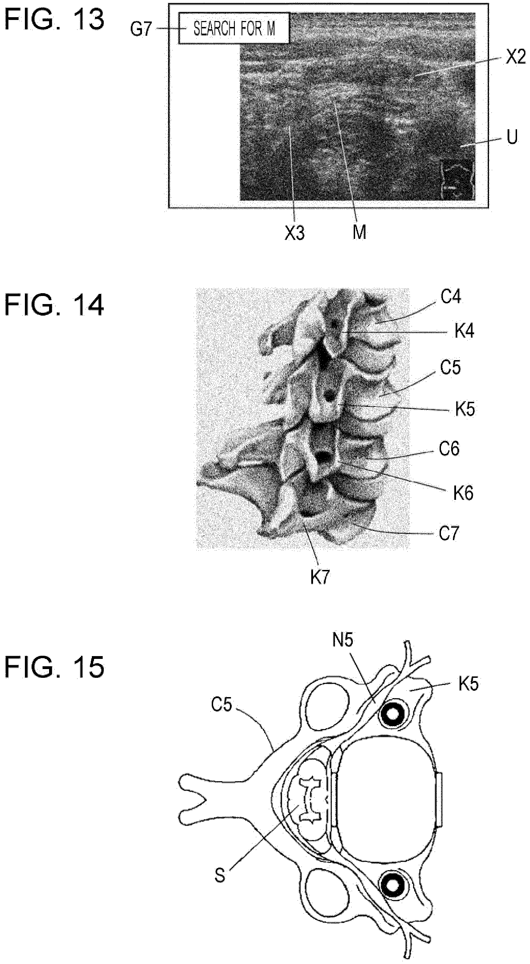

If the operation guide unit 10 determines that the peripheral site C has been detected, for example, as illustrated in Fig. 13, the operation guide unit 10 guides the user to search for the target site M, which is a long-axis view of the appendix. The long-axis view of the appendix represents a longitudinal cross-sectional image of the cross section of the appendix taken along the central axis of the appendix. At this time, the operation guide unit 10 can display a guide marker G7 to search for the peripheral site C on the display unit 7 together with the ultrasound image U. The operation guide unit 10 can provide a specific instruction to the user, such as displaying in the guide marker G7 a message that prompts the user to move the ultrasound probe 15 downward with the attitude of the ultrasound probe 15 maintained to acquire a tomographic image including the target site M.

When a guide to search for the target site M is provided in this way, the site recognition unit 9 performs the detection process of the target site M, and the operation guide unit 10 determines whether the target site M has been detected. At this time, for example, as illustrated in Fig. 13, the site recognition unit 9 can recognize the long-axis view of the ileum as an auxiliary site X2, recognize the long-axis view of the cecum as an auxiliary site X3, and detect the target site M in consideration of these recognition results. If the target site M has not been detected, the operation guide unit 10 provides a guide to search for the target site M, and the site recognition unit 9 performs the detection process of the target site M. In this way, a guiding process using the operation guide unit 10 and the detection process of the target site M using the site recognition unit 9 are repeatedly performed until the target site M is detected.

If the operation guide unit 10 determines that the target site M has been detected, the operation guide unit 10 notifies the user that the cross section of the target site M is displayed on the display unit 7. Then, the operation of the ultrasound diagnostic apparatus 1 for detecting the appendix, which is the target site M, ends.

As described above, even when the target site M, for example, the appendix, which is different from the common bile duct, is to be detected, as in Embodiment 1, a user is guided to operate the ultrasound probe 15 so as to detect the peripheral sites effective to detect the target site M, and the user is guided to operate the ultrasound probe 15 so as to detect the target site M on the basis of the recognition result of the peripheral sites. This eliminates the need to perform an unnecessary image recognition process and enables easy and rapid detection of the target site M with the reduced calculation load on the ultrasound diagnostic apparatus 1.

### Embodiment 3

In the neck of the human body, seven cervical vertebrae, including the first cervical vertebra to the seventh cervical vertebra, exist from the head side, and the spinal cord runs through the first cervical vertebra to the seventh cervical vertebra. Further, a plurality of nerves extend from the spinal cord through each of the seven cervical vertebrae. Nerve roots of the nerves are generally observed using an ultrasound diagnostic apparatus during the diagnosis of a disease or the like, such as in the practice of so-called nerve blocks and the like. Among the nerve roots of the first cervical vertebra to the seventh cervical vertebra, the nerve roots of the fifth cervical vertebra to the seventh cervical vertebra run in parallel in such a manner as to be adjacent to each other, and thus it is generally difficult for a less experienced user to identify the nerve roots of the fifth cervical vertebra to the seventh cervical vertebra by observing an ultrasound image.

Furthermore, it is known that a long-axis view of the nerve root of the sixth cervical vertebra is relatively easily depicted among the nerve roots of the fifth cervical vertebra to the seventh cervical vertebra. Accordingly, Embodiment 3 introduces the operation of the ultrasound diagnostic apparatus 1 for detecting the nerve root of the sixth cervical vertebra as a peripheral site and the nerve root of fifth cervical vertebra and the nerve root of the seventh cervical vertebra as target sites.

The long-axis view of the nerve root of the sixth cervical vertebra represents a longitudinal cross-sectional image of the cross section of the nerve root of the sixth cervical vertebra taken along the central axis of the nerve root of the sixth cervical vertebra.

Fig. 14 schematically illustrates a fifth cervical vertebra C5, a sixth cervical vertebra C6, and a seventh cervical vertebra C7. The fifth cervical vertebra C5, the sixth cervical vertebra C6, and the seventh cervical vertebra C7 have transverse processes K5, K6, and K.7, respectively. Further, as illustrated in Fig. 15, a nerve root N5 of the fifth cervical vertebra C5 extends from a spinal cord S along the transverse process K5 of the fifth cervical vertebra C5. Like the fifth cervical vertebra C5, a nerve root N6 of the sixth cervical vertebra C6 extends from the spinal cord S along the transverse process K6 of the sixth cervical vertebra C6, and a nerve root N7 of the seventh cervical vertebra C7 extends from the spinal cord S along the transverse process K7 of the seventh cervical vertebra C7, although not illustrated.

The peripheral site memory 11 of the ultrasound diagnostic apparatus 1 is assumed to store a long-axis view of the nerve root N6 of the sixth cervical vertebra C6 as a peripheral site effective to detect the nerve root N5 of the fifth cervical vertebra C5 and the nerve root N7 of the seventh cervical vertebra C7, and further store a detection order such that the nerve root N6 of the sixth cervical vertebra C6, the nerve root N5 of the fifth cervical vertebra C5, and the nerve root N7 of the seventh cervical vertebra C7 are detected in this order.

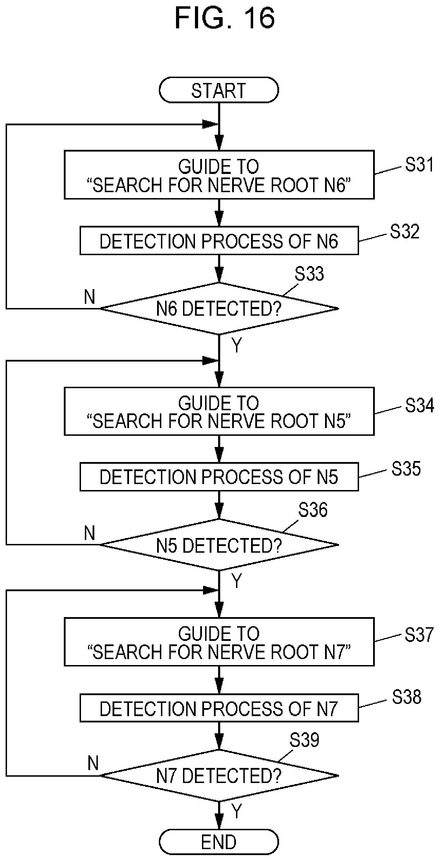

Fig. 16 is a flowchart illustrating the operation of the ultrasound diagnostic apparatus 1 according to Embodiment 3.

First, in step S31, the operation guide unit 10 guides a user to search for the long-axis view of the nerve root N6 of the sixth cervical vertebra C6 as a peripheral site. At this time, for example, the operation guide unit 10 can display a guide marker to search for the nerve root N6 of the sixth cervical vertebra C6 on the display unit 7 together with the ultrasound image U. This guide marker may include a text, or may include a guide image representing a guide for the user.

When a guide to search for the nerve root N6 of the sixth cervical vertebra C6 is provided, the user operates the ultrasound probe 15 so that the nerve root N6 of the sixth cervical vertebra C6 is detected in accordance with the guide provided by the operation guide unit 10. In this way, in the state where the ultrasound probe 15 is being operated by the user, in step S32, the site recognition unit 9 performs the detection process of the nerve root N6 of the sixth cervical vertebra C6. The long-axis view of the nerve root N6 of the sixth cervical vertebra C6 is known to be depicted together adjoining the vertebral artery. For this reason, when performing the detection process of the nerve root of the sixth cervical vertebra C6, for example, the site recognition unit 9 also performs the detection process of the vertebral artery, and in response to the detection of the vertebral artery depicted together adjoining a long-axis view of a nerve root, the nerve root can be detected as the nerve root N6 of the sixth cervical vertebra C6. The site recognition unit 9 is capable of detecting the nerve root N6 of the sixth cervical vertebra C6 and the vertebral artery, which are depicted in the ultrasound image U, by using so-called template matching or the like.

Then, in step S33, the operation guide unit 10 determines whether the nerve root N6 of the sixth cervical vertebra C6 has been detected. If it is determined in step S33 that the nerve root N6 of the sixth cervical vertebra C6 has not been detected, the process returns to step S31, and the operation guide unit 10 again provides a guide to search for the nerve root N6 of the sixth cervical vertebra C6. Then, in step S32, the site recognition unit 9 performs the detection process of the nerve root N6 of the sixth cervical vertebra C6. In this way, the processing of steps S31 to S33 is repeatedly performed until the nerve root N6 of the sixth cervical vertebra C6 is detected.

If the operation guide unit 10 determines in step S33 that the nerve root N6 of the sixth cervical vertebra C6 has been detected, the process proceeds to step S34. In step S34, the operation guide unit 10 guides the user to search for a long-axis view of the nerve root N5 of the fifth cervical vertebra C5 as a target site. At this time, the operation guide unit 10 can display a guide marker to search for a long-axis view of the nerve root N5 of the fifth cervical vertebra C5 on the display unit 7 together with the ultrasound image U. The operation guide unit 10 can provide a specific instruction to the user, such as displaying in the guide marker a message that prompts the user to move the ultrasound probe 15 upward, that is, move the ultrasound probe 15 to the head side of the subject, to acquire a tomographic image including the nerve root N5 of the fifth cervical vertebra C5. The long-axis view of the nerve root N5 of the fifth cervical vertebra C5 represents a longitudinal cross-sectional image of the cross section of the nerve root N5 of the fifth cervical vertebra C5 taken along the central axis of the nerve root N5 of the fifth cervical vertebra C5.

When a guide to search for the long-axis view of the nerve root N5 of the fifth cervical vertebra C5 is provided in step S34, the user operates the ultrasound probe 15 so that the long-axis view of the nerve root N5 of the fifth cervical vertebra C5 is depicted in accordance with the guide provided by the operation guide unit 10. In this manner, in the state where the ultrasound probe 15 is being operated by the user, in step S35, the site recognition unit 9 performs the detection process of the nerve root N5 of the fifth cervical vertebra C5. Since the nerve root N5 of the fifth cervical vertebra C5, the nerve root N6 of the sixth cervical vertebra C6, and the nerve root N7 of the seventh cervical vertebra C7 have similar shapes, the site recognition unit 9 detects a shape resembling a long-axis view of a nerve root existing in the ultrasound image U as the long-axis view of the nerve root N5 of the fifth cervical vertebra C5.

Then, in step S36, the operation guide unit 10 determines whether the long-axis view of the nerve root N5 of the fifth cervical vertebra C5 has been detected. If it is determined in step S36 that the long-axis view of the nerve root N5 of the fifth cervical vertebra C5 has not been detected, the process returns to step S34, and the operation guide unit 10 again provides a guide to search for the nerve root N5 of the fifth cervical vertebra C5. Then, in step S35, the site recognition unit 9 performs the detection process of the nerve root N5 of the fifth cervical vertebra C5. In this way, the processing of steps S34 to S36 is repeatedly performed until the long-axis view of the nerve root N5 of the fifth cervical vertebra C5 is detected.

If it is determined in step S36 that the long-axis view of the nerve root N5 of the fifth cervical vertebra C5 has been detected, the process proceeds to step S37. In step S37, the operation guide unit 10 guides the user to search for a long-axis view of the nerve root N7 of the seventh cervical vertebra C7 as a target site. At this time, the operation guide unit 10 can display a guide marker to search for a long-axis view of the nerve root N7 of the seventh cervical vertebra C7 on the display unit 7 together with the ultrasound image U. The operation guide unit 10 can provide a specific instruction to the user, such as displaying in the guide marker a message that prompts the user to move the ultrasound probe 15 downward, that is, move the ultrasound probe 15 to the torso side of the subject, to acquire a tomographic image including the nerve root N7 of the seventh cervical vertebra C7. The long-axis view of the nerve root N7 of the seventh cervical vertebra C7 represents a longitudinal cross-sectional image of the cross section of the nerve root N7 of the seventh cervical vertebra C7 taken along the central axis of the nerve root N7 of the seventh cervical vertebra C7.

When a guide to search for the long-axis view of the nerve root N7 of the seventh cervical vertebra C7 is provided in step S37, the user operates the ultrasound probe 15 so that the long-axis view of the nerve root N7 of the seventh cervical vertebra C7 is depicted in accordance with the guide provided by the operation guide unit 10. In this manner, in the state where the ultrasound probe 15 is being operated by the user, in step S38, the site recognition unit 9 performs the detection process of the nerve root N7 of the seventh cervical vertebra C7. At this time, like the detection of the nerve root N5 of the fifth cervical vertebra C5, the site recognition unit 9 detects a shape resembling a long-axis view of a nerve root as the nerve root N7 of the seventh cervical vertebra C7.

Then, in step S39, the operation guide unit 10 determines whether the long-axis view of the nerve root N7 of the seventh cervical vertebra C7 has been detected. If it is determined in step S39 that the long-axis view of the nerve root N7 of the seventh cervical vertebra C7 has not been detected, the process returns to step S37, and the operation guide unit 10 again provides a guide to search for the nerve root N7 of the seventh cervical vertebra C7. Then, in step S38, the site recognition unit 9 performs the detection process of the nerve root N7 of the seventh cervical vertebra C7. In this way, the processing of steps S37 to S39 is repeatedly performed until the long-axis view of the nerve root N7 of the seventh cervical vertebra C7 is detected. If it is determined in step S39 that the long-axis view of the nerve root N7 of the seventh cervical vertebra C7 has been detected, the operation of the ultrasound diagnostic apparatus according to Embodiment 3 ends.

In the related art, the user observes an acquired ultrasound image to identify the nerve root N5 of the fifth cervical vertebra C5, the nerve root N6 of the sixth cervical vertebra C6, and the nerve root N7 of the seventh cervical vertebra C7. Since the nerve root N5 of the fifth cervical vertebra C5, the nerve root N6 of the sixth cervical vertebra C6, and the nerve root N7 of the seventh cervical vertebra C7 run in parallel in such a manner as to be adjacent to each other, it is generally difficult for a less experienced user to identify these nerve roots in an ultrasound image. However, as described above, the user is guided to operate the ultrasound probe 15 so as to search for the nerve root N5 of the fifth cervical vertebra C5 and the nerve root N7 of the seventh cervical vertebra C7 on the basis of the detection result of the nerve root N6 of the sixth cervical vertebra C6 in such a manner that the nerve root N6 of the sixth cervical vertebra C6 is used as a peripheral site and the nerve root N5 of the fifth cervical vertebra C5 and the nerve root N7 of the seventh cervical vertebra C7 are used as target sites. This enables easy and rapid detection of the nerve root N5 of the fifth cervical vertebra C5 and the nerve root N7 of the seventh cervical vertebra C7, regardless of the degree of experience of the user.

As illustrated in Fig. 14, the transverse process K5 of the fifth cervical vertebra C5, the transverse process K6 of the sixth cervical vertebra C6, and the transverse process K7 of the seventh cervical vertebra C7 have anatomically different shapes. In Embodiment 3, the site recognition unit 9 detects shapes resembling long-axis views of nerve roots to detect a long-axis view of the nerve root N5 of the fifth cervical vertebra C5 and the nerve root N7 of the seventh cervical vertebra C7. The shape of a transverse process existing around a detected nerve root can be used to check whether the detected nerve root is the nerve root N5 of the fifth cervical vertebra C5 or the nerve root N7 of the seventh cervical vertebra C7.

In this case, for example, in response to the long-axis view of the nerve root N5 of the fifth cervical vertebra C5 being detected in step S36 as a trigger, the operation guide unit 10 guides the user to search for a short-axis view of the nerve root N5 of the fifth cervical vertebra C5. At this time, the operation guide unit 10 can display a guide marker to search for a short-axis view of the nerve root N5 of the fifth cervical vertebra C5 on the display unit 7 together with the ultrasound image U. The operation guide unit 10 can provide a specific instruction to the user, such as displaying in the guide marker a message that prompts the user to rotate the orientation of the ultrasound probe 15 by 90 degrees to acquire a tomographic image including a short-axis view of the nerve root N5 of the fifth cervical vertebra C5. The short-axis view of the nerve root N5 of the fifth cervical vertebra C5 represents a transverse cross-sectional image of the cross section of the nerve root N5 of the fifth cervical vertebra C5 taken along a plane perpendicular to the central axis of the nerve root N5 of the fifth cervical vertebra C5.

When a guide to search for the short-axis view of the nerve root N5 of the fifth cervical vertebra C5 is provided, the user operates the ultrasound probe 15 so that the short-axis view of the nerve root N5 of the fifth cervical vertebra C5 is depicted in accordance with the guide provided by the operation guide unit 10. In this manner, in the state where the ultrasound probe 15 is being operated by the user, the site recognition unit 9 performs the detection process of the short-axis view of the nerve root N5 of the fifth cervical vertebra C5. At this time, the site recognition unit 9 performs processing to detect a transverse process existing around a nerve root. When the shape of the transverse process detected together with the nerve root is a shape specific to the transverse process K5 of the fifth cervical vertebra C5, the site recognition unit 9 can detect the detected nerve root as the nerve root N5 of the fifth cervical vertebra C5.

Further, for example, in response to a long-axis view of the nerve root N7 of the seventh cervical vertebra C7 being detected in step S39 as a trigger, the operation guide unit 10 guides the user to search for a short-axis view of the nerve root N7 of the seventh cervical vertebra C7. At this time, the operation guide unit 10 can display a guide marker to search for a short-axis view of the nerve root N7 of the seventh cervical vertebra C7 on the display unit 7 together with the ultrasound image U. The operation guide unit 10 can provide a specific instruction to the user, such as displaying in the guide marker a message that prompts the user to rotate the orientation of the ultrasound probe 15 by 90 degrees to acquire a tomographic image including a short-axis view of the nerve root N7 of the seventh cervical vertebra C7. The short-axis view of the nerve root N7 of the seventh cervical vertebra C7 represents a transverse cross-sectional image of the cross section of the nerve root N7 of the seventh cervical vertebra C7 taken along a plane perpendicular to the central axis of the nerve root N7 of the seventh cervical vertebra C7.

When a guide to search for the short-axis view of the nerve root N7 of the seventh cervical vertebra C7 is provided, the user operates the ultrasound probe 15 so that the short-axis view of the nerve root N7 of the seventh cervical vertebra C7 is depicted in accordance with the guide provided by the operation guide unit 10. In this manner, in the state where the ultrasound probe 15 is being operated by the user, the site recognition unit 9 performs the detection process of the short-axis view of the nerve root N7 of the seventh cervical vertebra C7. At this time, the site recognition unit 9 performs processing to detect the shape of a vertebra existing around a nerve root. When the shape of the transverse process detected together with the nerve root is a shape specific to the transverse process K7 of the seventh cervical vertebra C7, the site recognition unit 9 can detect the detected nerve root as the nerve root N7 of the seventh cervical vertebra C7.

In this way, the site recognition unit 9 detects the shape of a vertebra existing around a nerve root, thereby enabling improvement in the detection accuracy of the nerve root N5 of the fifth cervical vertebra C5 and the nerve root N7 of the seventh cervical vertebra C7.

Furthermore, during the detection process of the nerve root N6 of the sixth cervical vertebra C6, if a vertebral artery is detected together with a long-axis view of a nerve root, the site recognition unit 9 detects this nerve root as the nerve root N6 of the sixth cervical vertebra C6. In addition, like the detection process of the nerve root N5 of the fifth cervical vertebra C5 and the nerve root N7 of the seventh cervical vertebra C7, the shape of a transverse process existing around a nerve root can be used to check whether the detected nerve root is the nerve root N6 of the sixth cervical vertebra C6. In this case, for example, in response to a long-axis view of the nerve root N6 of the sixth cervical vertebra C6 being detected as a trigger, first, a guide to search for a short-axis view of the nerve root N6 of the sixth cervical vertebra C6 is provided, and, further, in response to a short-axis view of the nerve root N6 of the sixth cervical vertebra C6 being detected as a trigger, a guide to search for a long-axis view of the nerve root N5 of the fifth cervical vertebra C5 is provided. The short-axis view of the nerve root N6 of the sixth cervical vertebra C6 represents a transverse cross-sectional image of the cross section of the nerve root N6 of the sixth cervical vertebra C6 taken along a plane perpendicular to the central axis of the nerve root N6 of the sixth cervical vertebra C6.

When the operation guide unit 10 provides a guide to search for the short-axis view of the nerve root N6 of the sixth cervical vertebra C6, the user operates the ultrasound probe 15 so that the short-axis view of the nerve root N6 of the sixth cervical vertebra C6 is depicted in accordance with the guide provided by the operation guide unit 10. In this manner, in the state where the ultrasound probe 15 is being operated by the user, the site recognition unit 9 performs the detection process of the nerve root N6 of the sixth cervical vertebra C6. When performing processing to detect the short-axis view of the nerve root N6 of the sixth cervical vertebra C6, the site recognition unit 9 performs processing to detect the shape of a transverse process existing around a nerve root. When the shape of the transverse process detected together with the nerve root is a shape specific to the transverse process K6 of the sixth cervical vertebra C6, the site recognition unit 9 can detect this nerve root as the nerve root N6 of the sixth cervical vertebra C6. This can improve the detection accuracy of the nerve root N6 of the sixth cervical vertebra C6, and thus the user is able to accurately search for the nerve root N5 of the fifth cervical vertebra C5 and the nerve root N7 of the seventh cervical vertebra C7.

Further, when detecting the nerve root N6 of the sixth cervical vertebra C6, the site recognition unit 9 detects the vertebral artery depicted together with the long-axis view of the nerve root of the sixth cervical vertebra C6 by using image analysis such as so-called template matching. Since blood flows in the vertebral artery, the vertebral artery can be detected based on a so-called Doppler signal.

Further, if a certain amount of time elapses without the long-axis view of the nerve root N5 of the fifth cervical vertebra C5 being detected after the operation guide unit 10 provides a guide to search for the long-axis view of the nerve root N5 of the fifth cervical vertebra C5, it is determined that the user is undecided where to move the ultrasound probe 15. Then, the operation guide unit 10 can guide the user to again depict the long-axis view of the nerve root N6 of the sixth cervical vertebra C6. Also, if a certain amount of time elapses without the long-axis view of the nerve root N7 of the seventh cervical vertebra C7 being detected after the operation guide unit 10 provides a guide to search for the long-axis view of the nerve root N7 of the seventh cervical vertebra C7, the operation guide unit 10 can guide the user to again depict the long-axis view of the nerve root N6 of the sixth cervical vertebra C6. In this way, guiding the user to again depict the long-axis view of the nerve root N6 of the sixth cervical vertebra C6 allows the user to easily depict the nerve root N5 of the fifth cervical vertebra C5 and the nerve root N7 of the seventh cervical vertebra C7 using the position of the nerve root N6 of the sixth cervical vertebra C6 as a reference.

The nerve root N5 of the fifth cervical vertebra C5, the nerve root N6 of the sixth cervical vertebra C6, and the nerve root N7 of the seventh cervical vertebra C7 are anatomically located at adjacent positions close to each other. For this reason, image patterns depicted in ultrasound images U sequentially acquired during a period from when the long-axis view of the nerve root N6 of the sixth cervical vertebra C6 is depicted to when the long-axis view of the nerve root N5 of the fifth cervical vertebra C5 is depicted or during a period from when the long-axis view of the nerve root N6 of the sixth cervical vertebra C6 is depicted to when the long-axis view of the nerve root N7 of the seventh cervical vertebra C7 is depicted are typically changed by a small amount. Accordingly, if the degree of similarity between an ultrasound image obtained at the point in time when the operation guide unit 10 provides a guide to search for the long-axis view of the nerve root N5 of the fifth cervical vertebra C5 or the long-axis view of the nerve root N7 of the seventh cervical vertebra C7 and a sequentially acquired ultrasound image U exceeds a determined threshold value, it is determined that the position of the ultrasound probe 15 is away from the nerve root N5 of the fifth cervical vertebra C5, the nerve root N6 of the sixth cervical vertebra C6, and the nerve root N7 of the seventh cervical vertebra C7. Then, the operation guide unit 10 can provide a guide to again depict the long-axis view of the nerve root N6 of the sixth cervical vertebra C6.

Further, the operation guide unit 10 guides the user to search for nerve roots in the order of the nerve root N6 of the sixth cervical vertebra C6, the nerve root N5 of the fifth cervical vertebra C5, and the nerve root N7 of the seventh cervical vertebra C7. Alternatively, the operation guide unit 10 may guide the user to search for nerve roots in the order of the nerve root N6 of the sixth cervical vertebra C6, the nerve root N7 of the seventh cervical vertebra C7, and the nerve root N5 of the fifth cervical vertebra C5. Also in this case, like guiding the user to search for nerve roots in the order of the nerve root N6 of the sixth cervical vertebra C6, the nerve root N5 of the fifth cervical vertebra C5, and the nerve root N7 of the seventh cervical vertebra C7, the nerve root N5 of the fifth cervical vertebra C5 and the nerve root N7 of the seventh cervical vertebra C7 can be easily and rapidly detected, regardless of the degree of experience of the user.

Further, the shape of the transverse process K5 of the fifth cervical vertebra C5 is detected to detect the short-axis view of the nerve root N5 of the fifth cervical vertebra C5, and the shape of the transverse process K7 of the seventh cervical vertebra C7 is detected to detect the nerve root N7 of the seventh cervical vertebra C7. Alternatively, the site recognition unit 9 may use a machine learning technique or a typical image recognition technique based on deep learning to classify the short-axis view of the nerve root N5 of the fifth cervical vertebra C5 and the short-axis view of the nerve root N7 of the seventh cervical vertebra C7. Likewise, the short-axis view of the nerve root N6 of the sixth cervical vertebra C6 can also be classified by using a machine learning technique or a typical image recognition technique based on deep learning.

### Embodiment 4

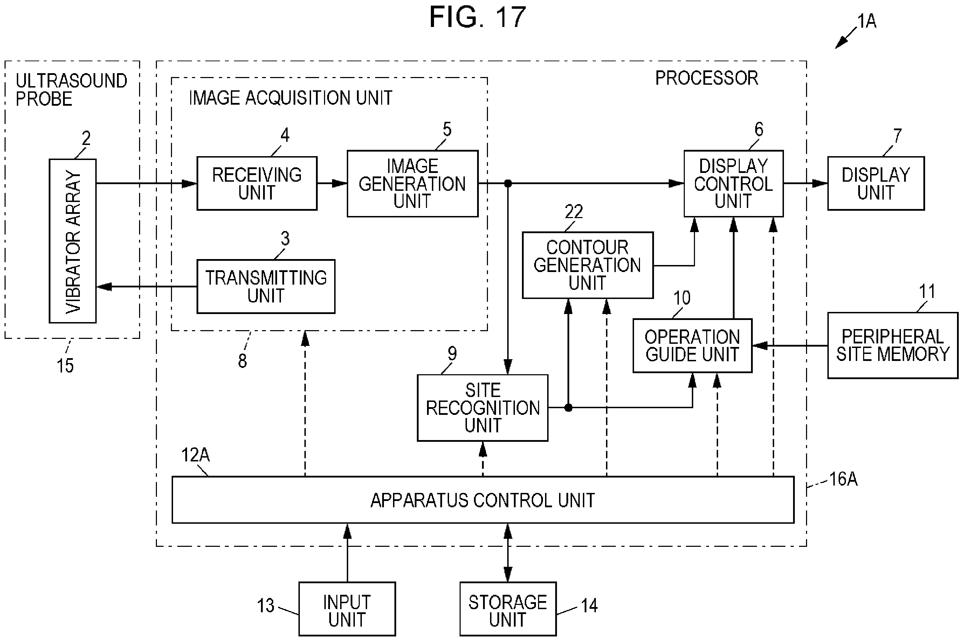

Fig. 17 illustrates a configuration of an ultrasound diagnostic apparatus 1A according to Embodiment 4. The ultrasound diagnostic apparatus 1A of Embodiment 4 includes an apparatus control unit 12A in place of the apparatus control unit 12 in the ultrasound diagnostic apparatus 1 of Embodiment 1 illustrated in Fig. 1, and additionally includes a contour generation unit 22. In the ultrasound diagnostic apparatus 1A, the site recognition unit 9 is connected to the contour generation unit 22, and the contour generation unit 22 is connected to the display control unit 6. The apparatus control unit 12A is connected to the display control unit 6, the image acquisition unit 8, the site recognition unit 9, the operation guide unit 10, the input unit 13, the storage unit 14, and the contour generation unit 22.

The display control unit 6, the image acquisition unit 8, the site recognition unit 9, the operation guide unit 10, the apparatus control unit 12A, and the contour generation unit 22 constitute a processor 16A.

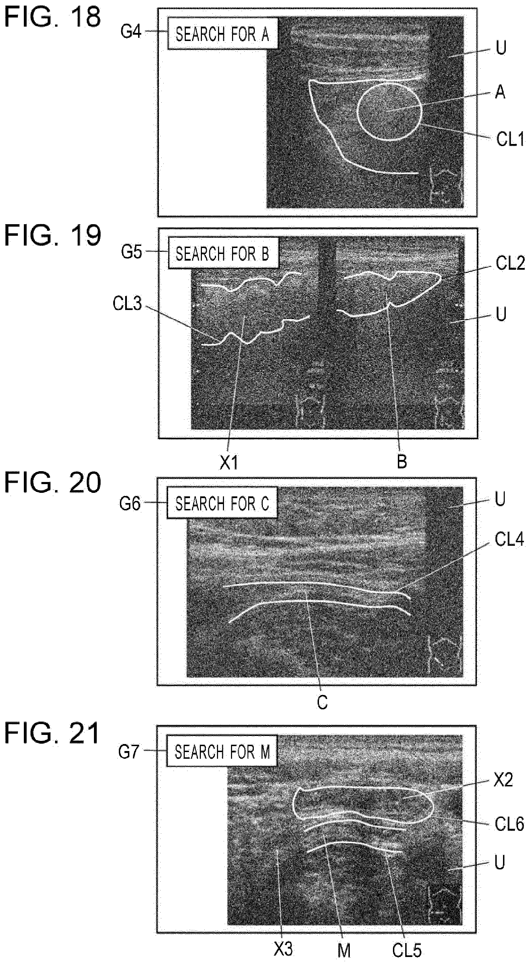

The contour generation unit 22 of the processor 16A generates contours of the peripheral sites, an auxiliary site, and the target site M, which are recognized by the site recognition unit 9, under control of the apparatus control unit 12A. The contours generated by the contour generation unit 22 in this manner are displayed superimposed on the ultrasound image U on the display unit 7 through the display control unit 6, as illustrated in Fig. 18 to Fig. 21, for example. In the example illustrated in Fig. 18, a contour line CL1 indicating the contour of the peripheral site A, which is the short-axis view of the ascending colon, is displayed superimposed on the ultrasound image U. In the example illustrated in Fig. 19, a contour line CL2 of the peripheral site B, which is the long-axis view of the cecum, and a contour line CL3 of an auxiliary site X1, which is the long-axis view of the ascending colon, are displayed superimposed on the ultrasound image U. In the example illustrated in Fig. 20, a contour line CL4 of the peripheral site C, which is the long-axis view of the ileum, is displayed superimposed on the ultrasound image U. In the example illustrated in Fig. 21, a contour line CL5 of the target site M, which is the long-axis view of the appendix, and a contour line CL6 of the auxiliary site X2, which is the long-axis view of the ileum, are displayed superimposed on the ultrasound image U.

In this manner, each time the site recognition unit 9 detects a peripheral site, an auxiliary site, and the target site M, the contour generation unit 22 generates contours of the detected peripheral site, auxiliary site, and target site and displays the contours in such a manner as to superimpose the contours on the ultrasound image U.

Accordingly, the ultrasound diagnostic apparatus 1A according to Embodiment 4 allows a user to easily understand the position of a peripheral site, an auxiliary site, and the target site M included in the ultrasound image U. This enables further easy detection of the peripheral site and the target site.

Embodiment 4 provides an example in which when the appendix is detected as the target site M, the contour generation unit 22 generates contours of the ascending colon, the cecum, the ileum, and the appendix. When a site different from the appendix, such as the common bile duct, is detected as the target site M, the contour generation unit 22 can also generate contours of a peripheral site, an auxiliary site, and the target site M in a similar way.

Further, the contour generation unit 22 may highlight the generated contours of the peripheral site, the auxiliary site, and the target site M on the display unit 7. For example, the contour generation unit 22 may display contour lines indicating the generated contours in color different from the color used for the ultrasound image U. Alternatively, for example, the contour generation unit 22 may display contour lines indicating the generated contours in a blinking manner. This allows the user to more clearly understand the peripheral site, the auxiliary site, and the target site M included in the ultrasound image U.

Further, the contour generation unit 22 may display areas indicating the peripheral site, the auxiliary site, and the target site M recognized by the site recognition unit 9, that is, areas defined by the generated contours, on the display unit 7 in color different from the color used for the ultrasound image U. This allows the user to further clearly understand the peripheral site, the auxiliary site, and the target site M included in the ultrasound image U.

### Embodiment 5

In Embodiments 1, 2, and 4, each of a plurality of peripheral sites effective to detect the target site M is detected. The detection of some peripheral sites among the plurality of peripheral sites may be skipped. An ultrasound diagnostic apparatus 1 according to Embodiment 5 has the same configuration as that of the ultrasound diagnostic apparatus 1 Embodiment 1 illustrated in Fig. 1.

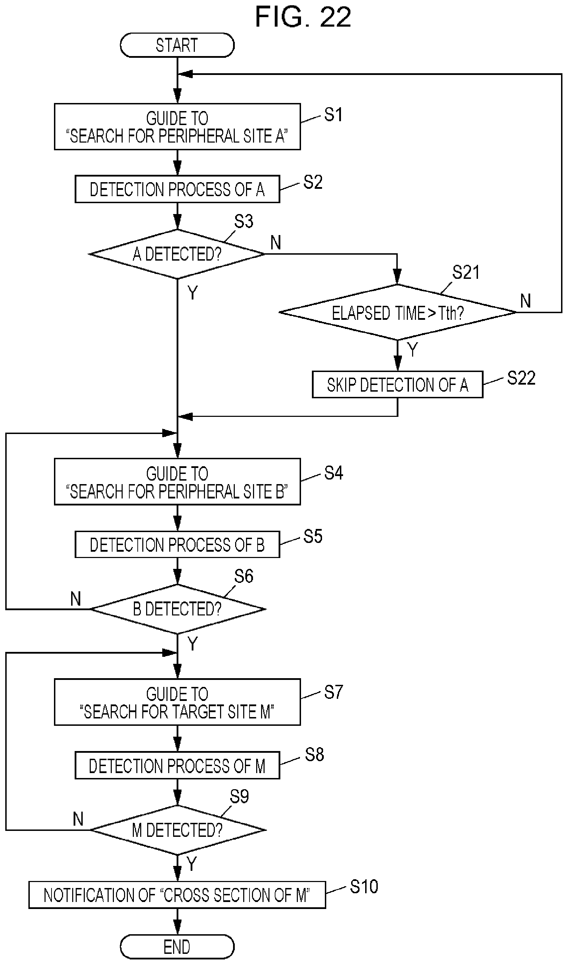

Fig. 22 is a flowchart illustrating the operation of the ultrasound diagnostic apparatus 1 according to Embodiment 5. In this flowchart, steps S1 to S10 are the same as steps S 1 to S10 in the flowchart illustrated in Fig. 4.

First, when the operation guide unit 10 provides a guide to search for the peripheral site A in step S1, then, in step S2, the site recognition unit 9 performs the detection process of the peripheral site A. Then, in step S3, the operation guide unit 10 determines whether the peripheral site A has been detected.

If the peripheral site A has not been detected, the process proceeds to step S21. In step S21, the operation guide unit 10 determines whether the elapsed time T since the point in time when a guide to search for the peripheral site A was provided in step S1 exceeds a threshold time Tth. If the elapsed time T is less than or equal to the threshold time Tth, the process returns to step S 1, and the operation guide unit 10 provides a guide to search for the peripheral site A. When the detection process of the peripheral site A is performed in step S2, then, in step S3, it is determined whether the peripheral site A has been detected. In this way, the processing of steps S1 to S3 and S21 is repeatedly performed so long as the peripheral site A remains undetected until the threshold time Tth elapses after a guide to search for the peripheral site A is provided in step S1.

If it is determined in step S21 that the elapsed time T exceeds the threshold time Tth, the process proceeds to step S22, in which the operation guide unit 10 skips the detection of the peripheral site A. Then, the process proceeds to step S4. The process also proceeds to step S4 if it is determined in step S3 that the peripheral site A has been detected.

When the operation guide unit 10 provides a guide to search for the peripheral site B in step S4, then, in step S5, the site recognition unit 9 performs the detection process of the peripheral site B. Then, in step S6, the operation guide unit 10 determines whether the peripheral site B has been detected. If the peripheral site B has not been detected, the process returns to step S4, and a guide to search for the peripheral site B is provided. Then, in step S5, the detection process of the peripheral site B is performed. In step S6, it is determined whether the peripheral site B has been detected. In this way, the processing of steps S4 to S6 is repeatedly performed until the peripheral site B is detected.

If it is determined in step S6 that the peripheral site B has been detected, the process proceeds to step S7. When the operation guide unit 10 provides a guide to search for the target site M in step S7, then, in step S8, the site recognition unit 9 performs the detection process of the target site M. Then, in step S9, the operation guide unit 10 determines whether the target site M has been detected. If the target site M has not been detected, the process returns to step S7, and a guide to search for the target site M is provided. Then, in step S8, the detection process of the target site M is performed. In step S9, it is determined whether the target site M has been detected. In this way, the processing of steps S7 to S9 is repeatedly performed until the target site M is detected.

If it is determined in step S9 that the target site M has been detected, the process proceeds to step S10, and the operation guide unit 10 notifies the user that the cross section of the target site M is displayed on the display unit 7. Then, the operation of the ultrasound diagnostic apparatus 1 according to Embodiment 5 ends.

As described above, if a peripheral site is not detectable until the threshold time Tth elapses after the operation guide unit 10 provides a guide, the ultrasound diagnostic apparatus 1 according to Embodiment 5 skips the detection of the peripheral site. This eliminates the need to perform a plurality of times a detection process of, for example, a peripheral site that is difficult to detect depending on the state of the subject or the like, and enables more rapid detection of the target site M.

In Embodiment 5, the detection process of the peripheral site A among the two peripheral sites A and B is skipped. Alternatively, the detection process of the peripheral site B, instead of the peripheral site A, may be skipped.

In Embodiment 5, furthermore, if a peripheral site is not detectable until the threshold time Tth elapses after the operation guide unit 10 provides a guide, the detection process of the peripheral site is skipped. Any other trigger for skipping the detection process of a peripheral site may be used.

For example, the operation guide unit 10 may skip the detection process of some peripheral sites among a plurality of peripheral sites effective to detect the target site M on the basis of the recognition result of a peripheral site, which is obtained by the site recognition unit 9. For example, the peripheral sites A and B are stored in the peripheral site memory 11 as peripheral sites effective to detect the target site M. In this case, in response to detection of the peripheral site A, the operation guide unit 10 can determine that there is no need to detect the peripheral site B, and can skip the detection process of the peripheral site B.

Alternatively, for example, the operation guide unit 10 may skip the detection process of some peripheral sites among a plurality of peripheral sites effective to detect the target site M on the basis of correction information input by the user through the input unit 13. For example, the user may input, as correction information, a peripheral site for which the detection process is to be skipped, through the input unit 13. For example, the peripheral sites A and B are stored in the peripheral site memory 11 as a plurality of peripheral sites effective to detect the target site M. In this case, in response to the user inputting information for skipping the peripheral site A as correction information through the input unit 13, the operation guide unit 10 skips the detection process of the peripheral site A.

Alternatively, for example, the operation guide unit 10 may skip the detection process of some peripheral sites among a plurality of peripheral sites effective to detect the target site M on the basis of subject information concerning the state of the subject, which is input by the user through the input unit 13. For example, a plurality of peripheral sites effective to detect the target site M include the gallbladder. In response to the user inputting information indicating that the subject remains in the postprandial state through the input unit 13, the operation guide unit 10 can determine that the gallbladder is in a contracted state different from a normal state, and can skip the detection process of the gallbladder.

### Embodiment 6

In Embodiment 5, the detection of some peripheral sites among a plurality of peripheral sites effective to detect the target site M is skipped. Alternatively, the detection order of the plurality of peripheral sites may be changed before a user is guided. An ultrasound diagnostic apparatus 1 according to Embodiment 6 has the same configuration as that of the ultrasound diagnostic apparatus 1 Embodiment 1 illustrated in Fig. 1.

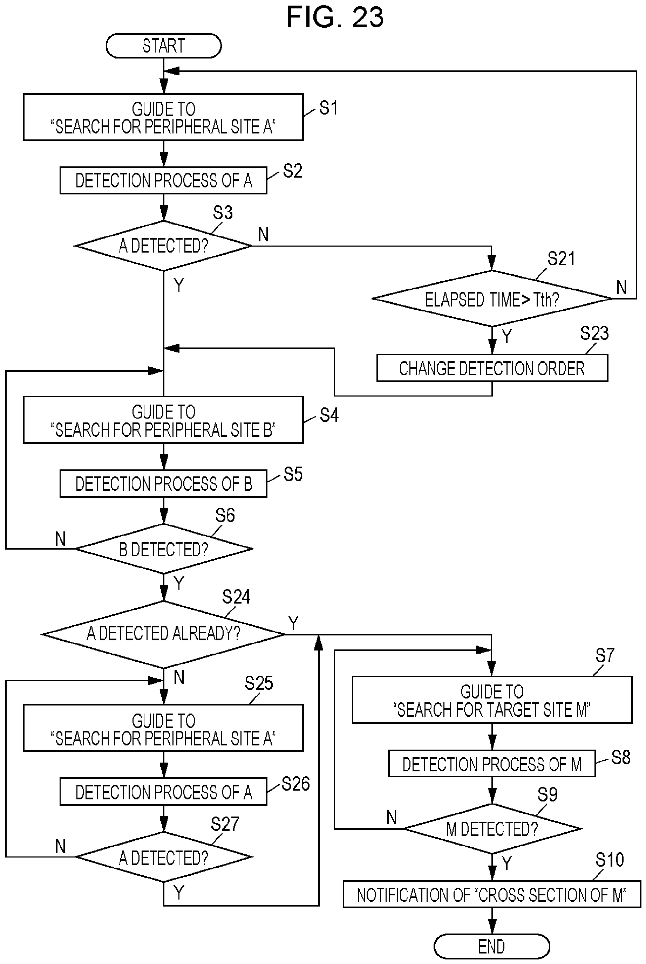

Fig. 23 is a flowchart illustrating the operation of the ultrasound diagnostic apparatus 1 according to Embodiment 6. In this flowchart, steps S1 to S10 are the same as steps S1 to S10 in the flowchart illustrated in Fig. 4, and step S21 is the same as step S21 in the flowchart illustrated in Fig. 22.

First, when the operation guide unit 10 provides a guide to search for the peripheral site A in step S 1, then, in step S2, the site recognition unit 9 performs the detection process of the peripheral site A. Then, in step S3, the operation guide unit 10 determines whether the peripheral site A has been detected.

If the peripheral site A has not been detected, the process proceeds to step S21. In step S21, the operation guide unit 10 determines whether the elapsed time T since the point in time when a guide to search for the peripheral site A was provided in step S1 exceeds a threshold time Tth. If the elapsed time T is less than or equal to the threshold time Tth, the process returns to step S 1, and the operation guide unit 10 provides a guide to search for the peripheral site A. When the detection process of the peripheral site A is performed in step S2, then, in step S3, it is determined whether the peripheral site A has been detected. In this way, the processing of steps S1 to S3 and S21 is repeatedly performed so long as the peripheral site A remains undetected until the threshold time Tth elapses after a guide to search for the peripheral site A is provided in step S 1.

If it is determined in step S21 that the elapsed time T exceeds the threshold time Tth, the process proceeds to step S23. In step S23, the operation guide unit 10 changes the detection order of the peripheral site A. For example, the operation guide unit 10 changes the detection order in which the peripheral site B is detected after the detection of the peripheral site A to the detection order in which the peripheral site A is detected after the detection of the peripheral site B. When the detection order of the peripheral site A is changed in step S21, the process proceeds to step S4. The process also proceeds to step S4 if it is determined in step S3 that the peripheral site A has been detected.

When the operation guide unit 10 provides a guide to search for the peripheral site B in step S4, then, in step S5, the site recognition unit 9 performs the detection process of the peripheral site B. Then, in step S6, the operation guide unit 10 determines whether the peripheral site B has been detected. If it is determined that the peripheral site B has not been detected, the process returns to step S4, and a guide to search for the peripheral site B is provided. Then, in step S5, the detection process of the peripheral site B is performed. In step S6, it is determined whether the peripheral site B has been detected. In this way, the processing of steps S4 to S6 is repeatedly performed until the peripheral site B is detected.

If it is determined in step S6 that the peripheral site B has been detected, the process proceeds to step S24. In step S24, the operation guide unit 10 determines whether the peripheral site A has already been detected in step S2. If it is determined that the peripheral site A has not yet been detected, it is determined that the detection of the peripheral site A has failed in step S3, and then the process proceeds to step S25.

The processing of step S25 is the same as the processing of step S1, and the operation guide unit 10 provides a guide to search for the peripheral site A. The subsequent processing of step S26 is the same as the processing of step S2, and the site recognition unit 9 performs the detection process of the peripheral site A. Since the detection of the peripheral site B has completed, for example, the site recognition unit 9 can perform the detection process of the peripheral site A in consideration of the recognition result of the peripheral site B. The processing of step S27 subsequent to step S26 is similar to the processing of step S3. In step S26, the operation guide unit 10 determines whether the peripheral site A has been detected.

If the peripheral site A has not been detected in step S27, the process returns to step S25, and a guide is provided to search for the peripheral site A. When the detection process of the peripheral site A is performed in step S26, then, in step S27, it is determined whether the peripheral site A has been detected. In this way, the processing of steps S25 to S27 is repeatedly performed until the peripheral site A is detected in step S26. If it is determined in step S27 that the peripheral site A has been detected, the process proceeds to step S7.

If it is determined in step S24 that the peripheral site A has already been detected, the process proceeds to step S7 without performing steps S25 to S27.

When the operation guide unit 10 provides a guide to search for the target site M in step S7, then, in step S8, the site recognition unit 9 performs the detection process of the target site M. Then, in step S9, the operation guide unit 10 determines whether the target site M has been detected. If the target site M has not been detected, the process returns to step S7, and a guide to search for the target site M is provided. Then, in step S8, the detection process of the target site M is performed. In step S9, it is determined whether the target site M has been detected. In this way, the processing of steps S7 to S9 is repeatedly performed until the target site M is detected.

If it is determined in step S9 that the target site M has been detected, the process proceeds to step S 10, and the operation guide unit 10 notifies the user that the cross section of the target site M is displayed on the display unit 7. Then, the operation of the ultrasound diagnostic apparatus 1 according to Embodiment 6 ends.

As described above, in the ultrasound diagnostic apparatus 1 according to Embodiment 6, if a peripheral site is not detectable until the threshold time Tth elapses after the operation guide unit 10 provides a guide, the detection order of the peripheral site is changed. This improves the detection accuracy of a peripheral site for which the detection order is changed in consideration of the recognition result of the detected peripheral site. Therefore, the ultrasound diagnostic apparatus 1 can easily and rapidly detect the target site M.

In Embodiment 6, if a peripheral site is not detectable until the threshold time Tth elapses after the operation guide unit 10 provides a guide, the detection order of the peripheral site is changed. Any other trigger for changing the detection order of a peripheral site may be used.

For example, the operation guide unit 10 may change the detection order of some peripheral sites among a plurality of peripheral sites effective to detect the target site M on the basis of the recognition result of a peripheral site, which is obtained by the site recognition unit 9, before guiding the user. For example, the peripheral site A, B, and C are stored in the peripheral site memory 11 as peripheral sites effective to detect the target site M such that the peripheral sites A, B, and C are detected in this order. In this case, in response to the detection of the peripheral site A, the operation guide unit 10 can determine that the detection of the peripheral site C is easier than the detection of the peripheral site B, and change the detection order of the peripheral sites A, B, and C to the order of the peripheral sites A, C, and B before guiding the user.

Alternatively, for example, the operation guide unit 10 may change the detection order of some peripheral sites among a plurality of peripheral sites effective to detect the target site M on the basis of correction information input by the user through the input unit 13, before guiding the user. For example, the user may input, as correction information, the detection order of the plurality of peripheral sites through the input unit 13. For example, the peripheral sites A and B are stored in the peripheral site memory 11 as peripheral sites effective to detect the target site M such that the peripheral sites A and B are detected in this order. In this case, in response to the user inputting correction information indicating that the peripheral site A is to be detected second and the peripheral site B is to be detected first, the detection order of the peripheral sites A and B can be changed to the order of the peripheral sites B and A before the user is guided.

Alternatively, for example, the operation guide unit 10 may change the detection order of some peripheral sites among a plurality of peripheral sites effective to detect the target site M on the basis of subject information concerning information on the subject, which is input by the user through the input unit 13, before guiding the user. For example, a plurality of peripheral sites effective to detect the target site M include the gallbladder. In response to the user inputting information indicating that the subject remains in the postprandial state through the input unit 13, the operation guide unit 10 can determine that the gallbladder is in a contracted state different from a normal state, and can change the detection order of the gallbladder to detect the gallbladder later so that the gallbladder can be detected in consideration of a recognition result of any other peripheral site.

### Embodiment 7

In Embodiment 1, when guiding a user to operate the ultrasound probe 15, the operation guide unit 10 displays the guide markers G1 to G3 illustrated in Fig. 6 to Fig. 9 on the display unit 7. The operation guide unit 10 may guide the user to operate the ultrasound probe 15 using any other style. For example, the operation guide unit 10 may guide the user to operate the ultrasound probe 15 by using audio.

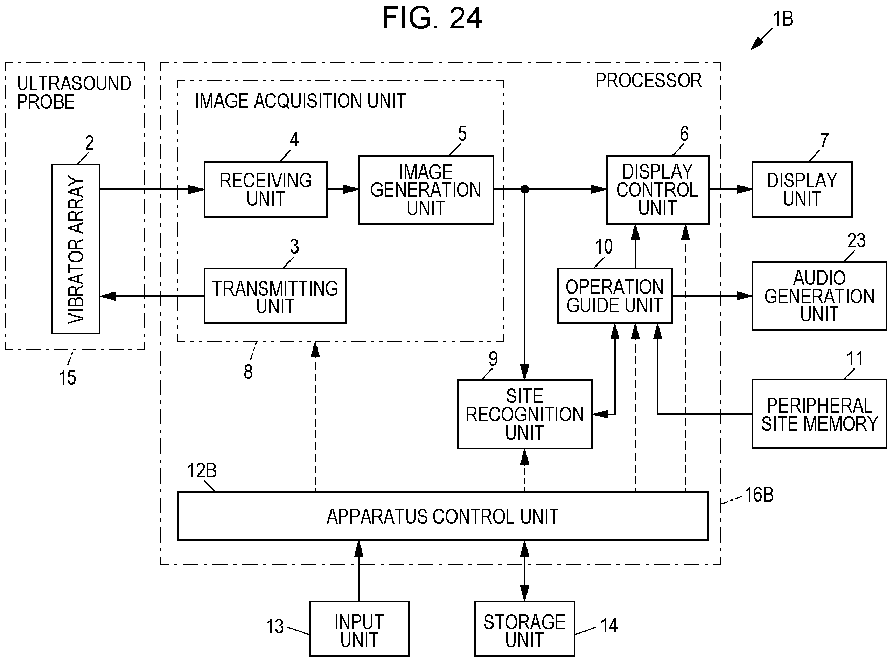

Fig. 24 is a block diagram illustrating a configuration of an ultrasound diagnostic apparatus 1B according to Embodiment 7. The ultrasound diagnostic apparatus 1B includes an apparatus control unit 12B in place of the apparatus control unit 12 in the ultrasound diagnostic apparatus 1 of Embodiment 1, and additionally includes an audio generation unit 23. In the ultrasound diagnostic apparatus 1B, the apparatus control unit 12B is connected to the display control unit 6, the image acquisition unit 8, the site recognition unit 9, the operation guide unit 10, the input unit 13, and the storage unit 14. The display control unit 6, the image acquisition unit 8, the site recognition unit 9, the operation guide unit 10, and the apparatus control unit 12B constitute a processor 16B.

The audio generation unit 23 is connected to the operation guide unit 10 of the processor 16B and is configured to include a speaker or the like to generate audio. This allows the operation guide unit 10 to guide the user to operate the ultrasound probe 15 by generating audio from the audio generation unit 23.

As described above, like the ultrasound diagnostic apparatus 1 according to Embodiment 1, the ultrasound diagnostic apparatus 1B according to Embodiment 7 guides a user to operate the ultrasound probe 15 so as to detect peripheral sites effective to detect the target site M, and guides the user to operate the ultrasound probe 15 so as to detect the target site M on the basis of the recognition result of the peripheral sites. This eliminates the need to perform an unnecessary image recognition process and enables easy and rapid detection of the target site M with the reduced calculation load on the ultrasound diagnostic apparatus 1.

While Embodiment 7 has been described as being applicable to Embodiment 1, Embodiment 7 is also applicable to Embodiments 2 to 6.

### Embodiment 8

The ultrasound diagnostic apparatus 1 of Embodiment 1 has a configuration in which the display unit 7 and the ultrasound probe 15 are connected directly to the processor 16. For example, the display unit 7, the ultrasound probe 15, and the processor 16 may be connected indirectly to each other via a network.

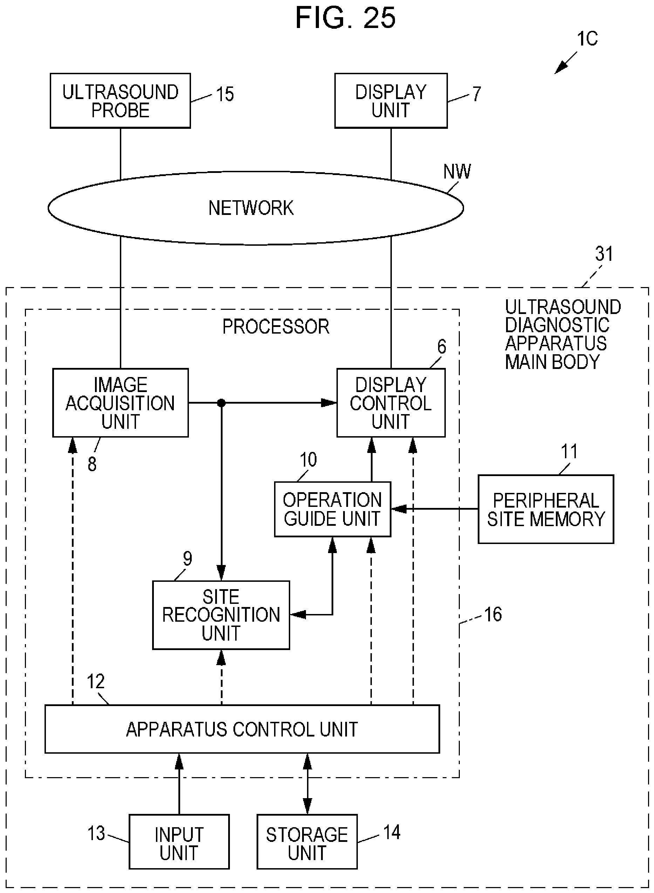

As illustrated in Fig. 25, an ultrasound diagnostic apparatus 1C according to Embodiment 8 is configured such that the display unit 7 and the ultrasound probe 15 are connected to an ultrasound diagnostic apparatus main body 31 via a network NW. The ultrasound diagnostic apparatus main body 31 is configured by removing the display unit 7 and the ultrasound probe 15 from the ultrasound diagnostic apparatus 1 of Embodiment 1 illustrated in Fig. 1. In the ultrasound diagnostic apparatus 31, the display control unit 6 and the image acquisition unit 8 are connected to the network NW.

When ultrasound beams are transmitted from the ultrasound probe 15 to the inside of the subject while the ultrasound probe 15 is pressed against the subject by the user, the vibrator array 2 of the ultrasound probe 15 receives ultrasound echoes reflected by the inside of the subject to generate reception signals. The ultrasound probe 15 transmits the generated reception signals to the ultrasound diagnostic apparatus main body 31 via the network NW.

The reception signals transmitted from the ultrasound probe 15 in the way described above are received by the image acquisition unit 8 of the ultrasound diagnostic apparatus main body 31 via the network NW, and the image acquisition unit 8 generates an ultrasound image in accordance with the reception signals.

The ultrasound image generated by the image acquisition unit 8 is sent to the display control unit 6 and the site recognition unit 9. The display control unit 6 performs predetermined processing on the ultrasound image sent from the image acquisition unit 8, and transmits the ultrasound image on which the predetermined processing is performed to the display unit 33 via the network NW. The ultrasound image transmitted from the display control unit 6 of the ultrasound diagnostic apparatus 31 in this way is received by the display unit 7 via the network NW and is displayed on the display unit 7.

Further, the site recognition unit 9 performs image analysis on the ultrasound image sent from the image acquisition unit 8 to recognize an imaged site of the subject, and detects a peripheral site or the target site M depicted in the ultrasound image.

In the detection of the target site M, the operation guide unit 10 guides the user to operate the ultrasound probe 15 so as to detect the peripheral site stored in the peripheral site memory 11, and further guides the user to operate the ultrasound probe 15 so as to detect the target site M on the basis of the recognition result of the site recognition unit 9. At this time, the operation guide unit 10 sends a text, an image, and the like indicating a guide for the user to the display control unit 6. The display control unit 6 transmits the text, image, and the like indicating the guide for the user to the display unit 7 via the network NW. The text, image, and the like indicating the guide for the user, which are sent from the display control unit 6, are received by the display unit 7 via the network NW and are displayed on the display unit 7.

As described above, the ultrasound diagnostic apparatus 1C according to Embodiment 8, in which the display unit 7 and the ultrasound probe 15 are connected to the ultrasound diagnostic apparatus main body 31 via the network NW, guides a user to operate the ultrasound probe 15 so as to detect a peripheral site effective to detect the target site M, and guides the user to operate the ultrasound probe 15 so as to detect the target site M on the basis of the recognition result of the peripheral site in a way similar to that for the ultrasound diagnostic apparatus 1 of the Embodiment 1. This eliminates the need to perform an unnecessary image recognition process, easily and rapidly detect the target site M.

Since the display unit 7 and the ultrasound probe 15 are connected to the ultrasound diagnostic apparatus main body 31 via the network NW, the ultrasound diagnostic apparatus main body 31 can be used as a so-called remote server. Accordingly, for example, if the user prepares only the display unit 7 and the ultrasound probe 15, the user can diagnose the subject. The usability of ultrasound diagnosis can be improved.

Furthermore, for example, when a portable thin computer called a tablet, or a display device or the like that is easily transportable is used as the display unit 7, the user can more easily perform ultrasound diagnosis of the subject. The usability of ultrasound diagnosis can further be improved.

While the display unit 7 and the ultrasound probe 15 are connected to the ultrasound diagnostic apparatus main body 31 via the network NW, the display unit 7, the ultrasound probe 15, and the ultrasound diagnostic apparatus main body 31 may be connected to the network NW via wired or wirelessly.

While Embodiment 8 has been described as being applicable to Embodiment 1, Embodiment 8 is also applicable to Embodiments 2 to 7. In particular, when Embodiment 8 is applied to Embodiment 7, the audio generation unit 23 in addition to the display unit 7 and the ultrasound probe 15 can be connected to the ultrasound diagnostic apparatus main body 31 via the network NW.

### Reference Signs List

- 1, 1A, 1B, 1C: ultrasound diagnostic apparatus
- 2: vibrator array
- 3: transmitting unit
- 4: receiving unit
- 5: image generation unit
- 6: display control unit
- 7: display unit
- 8: image acquisition unit
- 9: site recognition unit
- 10: operation guide unit
- 11: peripheral site memory
- 12: apparatus control unit
- 13: input unit
- 14: storage unit
- 15: ultrasound probe
- 16: processor
- 17: amplification unit
- 18: AD conversion unit
- 19: signal processing unit
- 20: DSC
- 21: image processing unit
- 22: contour generation unit
- 23: audio generation unit
- 31: ultrasound diagnostic apparatus main body
- A1, A2, B, C: peripheral site
- C5: fifth cervical vertebra
- C6: sixth cervical vertebra
- C7: seventh cervical vertebra
- CL1, CL2, CL3, CL4, CL5, CL6: contour line
- G1, G2, G3, G4, G5, G6: guide marker
- K5, K6, K7: transverse process
- M: target site
- N5, N6, N7: nerve root
- NW: network
- S: spinal cord
- T: elapsed time
- Tth: threshold time
- U: ultrasound image
- X, X1, X2: auxiliary site

## Claims

1. An ultrasound diagnostic apparatus (1) comprising:
an ultrasound probe (15);
an image acquisition unit (8) that transmits an ultrasound beam from the ultrasound probe (15) to a subject to acquire an ultrasound image (U);
a site recognition unit (9) that performs image analysis on the ultrasound image (U) acquired by the image acquisition unit (8) to recognize an imaged site of the subject;
a peripheral site memory (11) that stores a plurality of peripheral sites effective to detect a target site and a determined detection order in which the plurality of peripheral sites are detected; and
an operation guide unit (10) that, during detection of the target site, guides a user to operate the ultrasound probe (15) so as to sequentially detect the plurality of peripheral sites stored in the peripheral site memory (11) in accordance with the determined detection order and guides the user to operate the ultrasound probe (15) so as to detect the target site on the basis of a recognition result obtained by the site recognition unit (9).

2. The ultrasound diagnostic apparatus (1) according to claim 1, wherein
the operation guide unit (10) guides the user to operate the ultrasound probe (15) so as to skip detection of some peripheral sites among the plurality of peripheral sites and detect a subsequent peripheral site on the basis of the recognition result obtained by the site recognition unit (9), or guides the user to operate the ultrasound probe (15) so as to change the determined detection order and detect the plurality of peripheral sites in the changed detection order.

3. The ultrasound diagnostic apparatus (1) according to claim 1, further comprising
an input unit (13) that allows the user to perform an input operation.

4. The ultrasound diagnostic apparatus according to claim 3, wherein
the operation guide unit (10) guides the user to operate the ultrasound probe (15) so as to skip detection of some peripheral sites among the plurality of peripheral sites and detect a subsequent peripheral site on the basis of correction information input by the user through the input unit (13), or guides the user to operate the ultrasound probe (15) so as to change the determined detection order and detect the plurality of peripheral sites in the changed detection order.

5. The ultrasound diagnostic apparatus (1) according to claim 3, wherein
the operation guide unit (10) guides the user to operate the ultrasound probe (15) so as to skip detection of some peripheral sites among the plurality of peripheral sites and detect a subsequent peripheral site on the basis of subject information concerning a state of the subject, which is input by the user through the input unit (13), or guides the user to operate the ultrasound probe (15) so as to change the determined detection order and detect the plurality of peripheral sites in the changed detection order.

6. The ultrasound diagnostic apparatus (1) according to claim 3, wherein
when a determined amount of time elapses after the operation guide unit (10) guides the user to operate the ultrasound probe (15) so as to detect one peripheral site among the plurality of peripheral sites before the one peripheral site is detected, the operation guide unit (10) guides the user to operate the ultrasound probe (15) so as to skip detection of the one peripheral site and detect a subsequent peripheral site, or guides the user to operate the ultrasound probe (15) so as to change the determined detection order and detect the plurality of peripheral sites in the changed detection order.

7. The ultrasound diagnostic apparatus (1) according to any one of claims 3 to 6, wherein
the site recognition unit (9) recognizes an imaged site of the subject on the basis of subject information concerning a state of the subject, which is input by the user through the input unit (13).

8. The ultrasound diagnostic apparatus (1) according to any one of claims 1 to 7, wherein
the peripheral site memory (11) stores, for each subject, the plurality of peripheral sites effective to detect the target site and the determined detection order, and
the operation guide unit (10) guides the user to operate the ultrasound probe so as to sequentially detect the plurality of peripheral sites stored for each subject in accordance with the determined detection order stored for the subject.

9. The ultrasound diagnostic apparatus (1) according to any one of claims 1 to 8, further comprising a display unit (7), wherein
the operation guide unit (10) displays on the display unit (7) a guide provided to the user to operate the ultrasound probe (15).

10. The ultrasound diagnostic apparatus (1) according to claim 9, further comprising
a contour generation unit (22) that generates a contour of the plurality of peripheral sites recognized by the site recognition unit (9), wherein
the display unit (7) displays the ultrasound image (U) acquired by the image acquisition unit (8), and
the contour of the plurality of peripheral sites generated by the contour generation unit (22) is displayed superimposed on the ultrasound image (U) displayed on the display unit (7).

11. The ultrasound diagnostic apparatus (1) according to any one of claims 1 to 8, further comprising an audio generation unit (23), wherein
the operation guide unit (10) guides the user to operate the ultrasound probe (15) by generating audio from the audio generation unit (23).

12. The ultrasound diagnostic apparatus (1) according to any one of claims 1 to 11, wherein
the target site is a common bile duct, and
the plurality of peripheral sites includes a portal vein and a gallbladder.

13. The ultrasound diagnostic apparatus (1) according to any one of claims 1 to 11, wherein
the target site is an appendix, and
the plurality of peripheral sites includes an ascending colon, a cecum, and an ileum.

14. A method for controlling an ultrasound diagnostic apparatus (1), comprising:
acquiring an ultrasound image (U) on the basis of a reception signal generated by transmission and reception of an ultrasound beam from an ultrasound probe (15) to a subject;
performing image analysis on the acquired ultrasound image (U) to recognize an imaged site of the subject;
during detection of a target site, guiding a user to operate the ultrasound probe (15) so as to sequentially detect a plurality of peripheral sites effective to detect the target site in accordance with a determined detection order, and guiding the user to operate the ultrasound probe (15) so as to detect the target site on the basis of a recognition result obtained by the site recognition unit (9).

15. A processor for an ultrasound diagnostic apparatus (1), wherein the processor is configured to:
acquire an ultrasound image (U) on the basis of a reception signal generated by transmission and reception of an ultrasound beam from an ultrasound probe (15) to a subject;
perform image analysis on the acquired ultrasound image (U) to recognize an imaged site of the subject;
during detection of a target site, guide a user to operate the ultrasound probe (15) so as to sequentially detect a plurality of peripheral sites effective to detect the target site in accordance with a determined detection order, and guide the user to operate the ultrasound probe (15) so as to detect the target site on the basis of a recognition result for the imaged site.

16. The processor for an ultrasound diagnostic apparatus (1) according to claim 15, wherein the processor is connected to the ultrasound probe (15) via a network (NW).

## Patentansprüche

1. Ultraschalldiagnosegerät (1), umfassend:
eine Ultraschallsonde (15);
eine Bildaufnahmeeinheit (8), die einen Ultraschallstrahl von der Ultraschallsonde (15) zu einem Subjekt überträgt, um ein Ultraschallbild (U) aufzunehmen;
eine Stellenerkennungseinheit (9), die Bildanalyse an dem von der Bildaufnahmeeinheit (8) aufgenommenen Ultraschallbild (U) durchführt, um eine abgebildete Stelle des Subjekts zu erkennen;
einen Speicher für periphere Stellen (11), der eine Vielzahl peripherer Stellen, die zum Erfassen einer Zielstelle wirksam sind, und eine bestimmte Erfassungsreihenfolge, in der die Vielzahl peripherer Stellen erfasst wird, speichert; und
eine Bedienungsanleitungseinheit (10), die während der Erfassung der Zielstelle einen Benutzer anleitet, die Ultraschallsonde (15) zu bedienen, um nacheinander die Vielzahl peripherer Stellen zu erfassen, die in dem Speicher für periphere Stellen (11) gemäß der bestimmten Erfassungsreihenfolge gespeichert sind, und den Benutzer anleitet, die Ultraschallsonde (15) zu bedienen, um die Zielstelle auf der Grundlage eines von der Stellenerkennungseinheit (9) erhaltenen Erkennungsergebnisses zu erfassen.

2. Ultraschalldiagnosegerät (1) nach Anspruch 1, wobei
die Bedienungsanleitungseinheit (10) den Benutzer anleitet, die Ultraschallsonde (15) zu bedienen, um die Erfassung einiger peripherer Stellen aus der Vielzahl peripherer Stellen zu überspringen und eine nachfolgende periphere Stelle auf der Grundlage des von der Stellenerkennungseinheit (9) erhaltenen Erkennungsergebnisses zu erfassen, oder den Benutzer anleitet, die Ultraschallsonde (15) zu bedienen, um die bestimmte Erfassungsreihenfolge zu ändern und die Vielzahl peripherer Stellen in der geänderten Erfassungsreihenfolge zu erfassen.

3. Ultraschalldiagnosegerät (1) nach Anspruch 1, weiter umfassend:
eine Eingabeeinheit (13), die es dem Benutzer ermöglicht, eine Eingabeoperation durchzuführen.

4. Ultraschalldiagnosegerät nach Anspruch 3, wobei
die Bedienungsanleitungseinheit (10) den Benutzer anleitet, die Ultraschallsonde (15) zu bedienen, um die Erfassung einiger peripherer Stellen aus der Vielzahl peripherer Stellen zu überspringen und eine nachfolgende periphere Stelle auf der Grundlage von Korrekturinformationen zu erfassen, die von dem Benutzer durch die Eingabeeinheit (13) eingegeben werden, oder den Benutzer anleitet, die Ultraschallsonde (15) zu bedienen, um die bestimmte Erfassungsreihenfolge zu ändern und die Vielzahl peripherer Stellen in der geänderten Erfassungsreihenfolge zu erfassen.

5. Ultraschalldiagnosegerät (1) nach Anspruch 3, wobei
die Bedienungsanleitungseinheit (10) den Benutzer anleitet, die Ultraschallsonde (15) zu bedienen, um die Erfassung einiger peripherer Stellen aus der Vielzahl peripherer Stellen zu überspringen und eine nachfolgende periphere Stelle auf der Grundlage von Subjektinformationen bezüglich eines Zustands des Subjekts zu erfassen, die von dem Benutzer durch die Eingabeeinheit (13) eingegeben werden, oder den Benutzer angeleitet, die Ultraschallsonde (15) zu bedienen, um die bestimmte Erfassungsreihenfolge zu ändern und die Vielzahl peripherer Stellen in der geänderten Reihenfolge zu erfassen.

6. Ultraschalldiagnosegerät (1) nach Anspruch 3, wobei,
wenn eine bestimmte Zeitspanne verstreicht, nachdem die Bedienungsanleitungseinheit (10) den Benutzer angeleitet hat, die Ultraschallsonde (15) zu bedienen, um eine periphere Stelle aus der Vielzahl peripherer Stellen zu erfassen, bevor die eine periphere Stelle erfasst wird, die Bedienungsanleitungseinheit (10) den Benutzer anleitet, die Ultraschallsonde (15) zu bedienen, um die Erfassung der einen peripheren Stelle zu überspringen und eine nachfolgende periphere Stelle zu erfassen, oder den Benutzer anleitet, die Ultraschallsonde (15) zu bedienen, um die bestimmte Erfassungsreihenfolgezu ändern und die Vielzahl peripherer Stellen in der geänderten Erfassungsreihenfolge zu erkennen.

7. Ultraschalldiagnosegerät (1) nach einem der Ansprüche 3 bis 6, wobei
die Stellenerkennungseinheit (9) eine abgebildete Stelle des Subjekts auf der Grundlage von Subjektinformationen bezüglich eines Zustands des Subjekts erkennt, die von dem Benutzer über die Eingabeeinheit (13) eingegeben werden.

8. Ultraschalldiagnosegerät (1) nach einem der Ansprüche 1 bis 7, wobei
der Speicher für periphere Stellen (11) für jedes Subjekt die Vielzahl peripherer Stellen, die zum Erfassen der Zielstelle wirksam sind, und die bestimmte Erfassungsreihenfolge speichert, und
die Bedienungsanleitungseinheit (10) den Benutzer anleitet, die Ultraschallsonde zu bedienen, um nacheinander die für jedes Subjekt gespeicherte Vielzahl peripherer Stellen gemäß der für das Subjekt gespeicherten bestimmten Erfassungsreihenfolge zu erfassen.

9. Ultraschalldiagnosegerät (1) nach einem der Ansprüche 1 bis 8, das weiter eine Anzeigeeinheit (7) umfasst, wobei
die Bedienungsanleitungseinheit (10) auf der Anzeigeeinheit (7) eine dem Benutzer bereitgestellte Anleitung zum Bedienen der Ultraschallsonde (15) anzeigt.

10. Ultraschalldiagnosegerät (1) nach Anspruch 9, weiter umfassend:
eine Konturerzeugungseinheit (22), die eine Kontur der Vielzahl peripherer Stellen erzeugt, die von der Stellenerkennungseinheit (9) erkannt werden, wobei
die Anzeigeeinheit (7) das von der Bildaufnahmeeinheit (8) aufgenommene Ultraschallbild (U) anzeigt, und
die von der Konturerzeugungseinheit (22) erzeugte Kontur der Vielzahl peripherer Stellen dem auf der Anzeigeeinheit (7) angezeigten Ultraschallbild (U) überlagert angezeigt wird.

11. Ultraschalldiagnosegerät (1) nach einem der Ansprüche 1 bis 8, das weiter eine Tonerzeugungseinheit (23) umfasst, wobei
die Bedienungsanleitungseinheit (10) den Benutzer durch Erzeugen von Ton von der Tonerzeugungseinheit (23) anweist, die Ultraschallsonde (15) zu bedienen.

12. Ultraschalldiagnosegerät (1) nach einem der Ansprüche 1 bis 11, wobei
die Zielstelle ein Hauptgallengang ist, und
die Vielzahl peripherer Stellen eine Pfortader und eine Gallenblase beinhaltet.

13. Ultraschalldiagnosegerät (1) nach einem der Ansprüche 1 bis 11, wobei
die Zielstelle ein Blinddarm ist, und
die Vielzahl peripherer Stellen einen aufsteigenden Dickdarm, ein Zökum und ein Ileum beinhaltet.

14. Verfahren zum Steuern eines Ultraschallschalldiagnosegerät (1), umfassend:
Aufnehmen eines Ultraschallbildes (U) auf der Grundlage eines Empfangssignals, das durch Senden und Empfangen eines Ultraschallstrahls von einer Ultraschallsonde (15) an ein Subjekt erzeugt wird;
Durchführen einer Bildanalyse des aufgenommenen Ultraschallbildes (U), um eine abgebildete Stelle des Subjekts zu erkennen;
Anleiten eines Benutzers während der Erfassung einer Zielstelle, die Ultraschallsonde (15) zu bedienen, um nacheinander eine Vielzahl peripherer Stellen, die zum Erfassen der Zielstelle wirksam sind, gemäß einer bestimmten Erfassungsreihenfolge zu erfassen, und Anleiten des Benutzers, die Ultraschallsonde (15) zu bedienen, um die Zielstelle auf der Grundlage eines von der Stellenerkennungseinheit (9) erhaltenen Erkennungsergebnisses zu erfassen.

15. Prozessor für ein Ultraschalldiagnosegerät (1), wobei der Prozessor dazu konfiguriert ist:
ein Ultraschallbild (U) auf der Grundlage eines Empfangssignals, das durch Senden und Empfangen eines Ultraschallstrahls von einer Ultraschallsonde (15) an ein Subjekt erzeugt wird, aufzunehmen;
Bildanalyse des aufgenommenen Ultraschallbildes (U) durchzuführen, um eine abgebildete Stelle des Subjekts zu erkennen;
während der Erfassung einer Zielstelle, einen Benutzers anzuleiten, die Ultraschallsonde (15) zu bedienen, um nacheinander eine Vielzahl peripherer Stellen, die zum Erfassen der Zielstelle wirksam sind, gemäß einer bestimmten Erfassungsreihenfolge zu erfassen, und den Benutzer anzuleiten, die Ultraschallsonde (15) zu bedienen, um die Zielstelle auf der Grundlage eines Erkennungsergebnisses für die abgebildete Stelle zu erfassen.

16. Prozessor für ein Ultraschalldiagnosegerät (1) nach Anspruch 15, wobei der Prozessor über ein Netzwerk (NW) mit der Ultraschallsonde (15) verbunden ist.

## Revendications

1. Appareil de diagnostic à ultrasons (1) comprenant :
une sonde à ultrasons (15) ;
une unité d'acquisition d'image (8) qui émet un faisceau ultrasonore à partir de la sonde à ultrasons (15) à un sujet pour acquérir une image ultrasonore (U) ;
une unité de reconnaissance de site (9) qui effectue une analyse d'image sur l'image ultrasonore (U) acquise par l'unité d'acquisition d'image (8) pour reconnaître un site imagé du sujet ;
une mémoire de site périphérique (11) qui stocke une pluralité de sites périphériques efficaces pour détecter un site cible et un ordre de détection déterminé dans lequel la pluralité de sites périphériques sont détectés ; et
une unité de guidage d'opération (10) qui, pendant la détection du site cible, guide un utilisateur pour faire fonctionner la sonde à ultrasons (15) de manière à détecter séquentiellement la pluralité de sites périphériques stockés dans la mémoire de site périphérique (11) conformément à l'ordre de détection déterminé et guide l'utilisateur pour faire fonctionner la sonde à ultrasons (15) de manière à détecter le site cible sur la base d'un résultat de reconnaissance obtenu par l'unité de reconnaissance de site (9).

2. Appareil de diagnostic à ultrasons (1) selon la revendication 1, dans lequel
l'unité de guidage d'opération (10) guide l'utilisateur pour faire fonctionner la sonde à ultrasons (15) de manière à ignorer la détection de certains sites périphériques parmi la pluralité de sites périphériques et à détecter un site périphérique ultérieur sur la base du résultat de reconnaissance obtenu par l'unité de reconnaissance de site (9), ou guide l'utilisateur pour faire fonctionner la sonde à ultrasons (15) de manière à modifier l'ordre de détection déterminé et à détecter la pluralité de sites périphériques dans l'ordre de détection modifié.

3. Appareil de diagnostic à ultrasons (1) selon la revendication 1, comprenant en outre
une unité d'entrée (13) qui permet à l'utilisateur d'effectuer une opération d'entrée.

4. Appareil de diagnostic à ultrasons selon la revendication 3, dans lequel
l'unité de guidage d'opération (10) guide l'utilisateur pour faire fonctionner la sonde à ultrasons (15) de manière à ignorer la détection de certains sites périphériques parmi la pluralité de sites périphériques et à détecter un site périphérique ultérieur sur la base d'informations de correction entrées par l'utilisateur via l'unité d'entrée (13), ou guide l'utilisateur pour faire fonctionner la sonde à ultrasons (15) de manière à modifier l'ordre de détection déterminé et à détecter la pluralité de sites périphériques dans l'ordre de détection modifié.

5. Appareil de diagnostic à ultrasons (1) selon la revendication 3, dans lequel
l'unité de guidage d'opération (10) guide l'utilisateur pour faire fonctionner la sonde à ultrasons (15) de manière à ignorer la détection de certains sites périphériques parmi la pluralité de sites périphériques et à détecter un site périphérique ultérieur sur la base d'informations de sujet concernant un état du sujet, qui est saisi par l'utilisateur via l'unité d'entrée (13), ou guide l'utilisateur pour faire fonctionner la sonde à ultrasons (15) de manière à modifier l'ordre de détection déterminé et à détecter la pluralité de sites périphériques dans l'ordre de détection modifié.

6. Appareil de diagnostic à ultrasons (1) selon la revendication 3, dans lequel
lorsqu'un laps de temps déterminé s'écoule après que l'unité de guidage d'opération (10) a guidé l'utilisateur pour faire fonctionner la sonde à ultrasons (15) de manière à détecter un site périphérique parmi la pluralité de sites périphériques avant que le site périphérique soit détecté, l'unité de guidage d'opération (10) guide l'utilisateur pour faire fonctionner la sonde à ultrasons (15) de manière à ignorer la détection du site périphérique et à détecter un site périphérique ultérieur, ou guide l'utilisateur pour faire fonctionner la sonde à ultrasons (15) de manière à modifier l'ordre de détection déterminé et détecter la pluralité de sites périphériques dans l'ordre de détection modifié.

7. Appareil de diagnostic à ultrasons (1) selon l'une quelconque des revendications 3 à 6, dans lequel
l'unité de reconnaissance de site (9) reconnaît un site imagé du sujet sur la base d'informations de sujet concernant un état du sujet, qui sont entrées par l'utilisateur via l'unité d'entrée (13).

8. Appareil de diagnostic à ultrasons (1) selon l'une quelconque des revendications 1 à 7, dans lequel
la mémoire de sites périphériques (11) stocke, pour chaque sujet, la pluralité de sites périphériques efficaces pour détecter le site cible et l'ordre de détection déterminé, et
l'unité de guidage d'opération (10) guide l'utilisateur pour faire fonctionner la sonde à ultrasons de manière à détecter séquentiellement la pluralité de sites périphériques stockés pour chaque sujet conformément à l'ordre de détection déterminé stocké pour le sujet.

9. Appareil de diagnostic à ultrasons (1) selon l'une quelconque des revendications 1 à 8, comprenant en outre une unité d'affichage (7), dans lequel
l'unité de guidage d'opération (10) affiche sur l'unité d'affichage (7) un guide fourni à l'utilisateur pour faire fonctionner la sonde à ultrasons (15).

10. Appareil de diagnostic à ultrasons (1) selon la revendication 9, comprenant en outre
une unité de génération de contour (22) qui génère un contour de la pluralité de sites périphériques reconnus par l'unité de reconnaissance de site (9), dans lequel
l'unité d'affichage (7) affiche l'image ultrasonore (U) acquise par l'unité d'acquisition d'image (8), et
le contour de la pluralité de sites périphériques générés par l'unité de génération de contour (22) est affiché en superposition sur l'image ultrasonore (U) affichée sur l'unité d'affichage (7).

11. Appareil de diagnostic à ultrasons (1) selon l'une quelconque des revendications 1 à 8, comprenant en outre une unité de génération audio (23), dans lequel
l'unité de guidage d'opération (10) guide l'utilisateur pour faire fonctionner la sonde à ultrasons (15) en générant de l'audio à partir de l'unité de génération audio (23).

12. Appareil de diagnostic à ultrasons (1) selon l'une quelconque des revendications 1 à 11, dans lequel
le site cible est une voie biliaire principale, et
la pluralité de sites périphériques incluent une veine porte et une vésicule biliaire.

13. Appareil de diagnostic à ultrasons (1) selon l'une quelconque des revendications 1 à 11, dans lequel
le site cible est un appendice, et
la pluralité de sites périphériques incluent un côlon ascendant, un caecum et un iléon.

14. Procédé de commande d'un appareil de diagnostic à ultrasons (1), le procédé comprenant :
l'acquisition d'une image ultrasonore (U) sur la base d'un signal de réception généré par l'émission et la réception d'un faisceau ultrasonore en provenance d'une sonde à ultrasons (15) vers un sujet ;
la réalisation d'une analyse d'image sur l'image à ultrasons acquise (U) pour reconnaître un site imagé du sujet ;
pendant la détection d'un site cible, le guidage d'un utilisateur pour faire fonctionner la sonde à ultrasons (15) de manière à détecter séquentiellement une pluralité de sites périphériques efficaces pour détecter le site cible conformément à un ordre de détection déterminé, et le guidage de l'utilisateur pour faire fonctionner l'échographie sonde (15) de manière à détecter le site cible sur la base d'un résultat de reconnaissance obtenu par l'unité de reconnaissance de site (9).

15. Processeur pour un appareil de diagnostic par ultrasons (1), dans lequel le processeur est configuré pour :
acquérir une image ultrasonore (U) sur la base d'un signal de réception généré par l'émission et la réception d'un faisceau ultrasonore en provenance d'une sonde à ultrasons (15) vers un sujet ;
réaliser une analyse d'image sur l'image échographique acquise (U) pour reconnaître un site imagé du sujet ;
pendant la détection d'un site cible, guider un utilisateur pour faire fonctionner la sonde à ultrasons (15) de manière à détecter séquentiellement une pluralité de sites périphériques efficaces pour détecter le site cible conformément à un ordre de détection déterminé, et guider l'utilisateur pour faire fonctionner la sonde à ultrasons (15) de manière à détecter le site cible sur la base d'un résultat de reconnaissance pour le site imagé.

16. Processeur pour un appareil de diagnostic à ultrasons (1) selon la revendication 15, dans lequel le processeur est connecté à la sonde à ultrasons (15) via un réseau (NW).
